(19) Europäisches Patentamt
European Patent Office
Office européen des brevets

(11) Publication number : **0 541 335 A1**

# (12) EUROPEAN PATENT APPLICATION

(21) Application number : **92310067.1**

(22) Date of filing : **04.11.92**

(51) Int. Cl.$^5$ : **C12N 15/62,** C12N 5/10

(30) Priority : **08.11.91 US 792507**

(43) Date of publication of application :
**12.05.93 Bulletin 93/19**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Donnelly, John J.**
**1505 Brierwood Road**
**Havertown, PA 19083 (US)**
Inventor : **Liu, Margaret A.**
**4 Cushman Road**
**Rosemont, PA 19010 (US)**
Inventor : **Friedman, Arthur**
**121 Froghollow Road**
**Churchville, PA 18966 (US)**
Inventor : **Marshall, Mark S.**
**1519 Spruce Court**
**Carmel, Indiana 46032 (US)**
Inventor : **Hawe, Linda A.**
**2610 Skippack Pike**
**Norristown, PA 19403 (US)**
Inventor : **Montgomery, Donna L.**
**9 Hickory Lane**
**Chalfont, PA 18914 (US)**
Inventor : **Oliff, Allen A.**
**1412 Florence Drive**
**Gwynedd Valley, PA 19437 (US)**
Inventor : **Shi, Xiao-Ping**
**536 Winthrop Road**
**Collegeville, PA 19426 (US)**
Inventor : **Ulmer, Jeffrey**
**128 Dolly Circle**
**Chalfont, PA 18914 (US)**

(74) Representative : **Thompson, John Dr. et al**
**Merck & Co., Inc. European Patent**
**Department Terlings Park Eastwick Road**
**Harlow, Essex CM20 2QR (GB)**

## (54) Recombinant DNA sequences and plasmids for cellular immunity vaccines from bacterial toxinantigen conjugates.

(57) Recombinant DNA sequences coding for hybrid proteins having two primary components. The first component is a modified bacterial toxin that has translocating ability, while the second component is a polypeptide or protein that is exogenous to an antigen-presenting cell. The hybrid has the ability to be internalized by an antigen-presenting cell, where the hybrid is subsequently processed and an antigenic segment of the hybrid presented on the surface of the antigen-presenting cell, where the segment elicits an immune response by cytotoxic T lymphocytes.

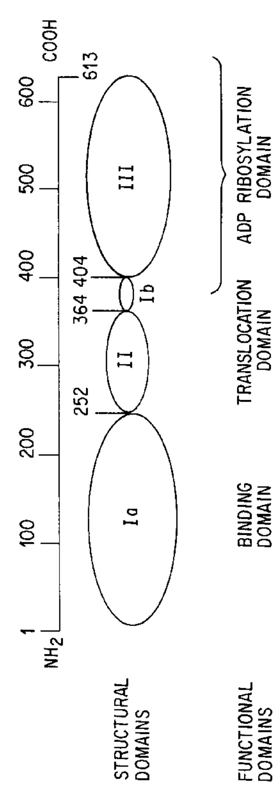


FIG. 1

EP 0 541 335 A1

## BACKGROUND OF THE INVENTION

The numerous substances and organisms that threaten the existence of animals having immune systems are either present in extracellular body fluids, such as toxins or bacteria, or else they are harbored within the animal's own cells, such as viruses, certain parasites and oncogene products. This distinction is important to thymusderived lymphocytes, also known as T cells, which are an important component of vertebrate immune systems. T cells have evolved parallel systems for recognizing intracellular and extracellular antigens. In both systems, antigens are recognized only when they are bound to molecules of the major histocompatability complex (MHC).

The MHC encodes two types of cell surface molecules that act as receptors for protein antigens. Class I MHC molecules consist of a highly polymorphic integral membrane glycoprotein alpha chain that is noncovalently bound to a $beta_2$ microglobulin. Class II MHC molecules consist of two noncovalently bound, highly polymorphic, integral membrane glycoproteins. Class I MHC molecules have a groove at the top surface formed by the two amino-terminal domains. The groove holds an antigen. As with other cell surface proteins, during cellular processing in the cytosol, MHC molecules are inserted into the endo-plasmic reticulum (ER) and, following chain assembly, are transported to the plasma membrane of the cell via the Golgi complex and post-Golgi complex vesicles.

The recognition of Class I vs. Class II molecules as antigen-presenting sites in general divides T cells into two classes, respectively termed cytotoxic T cells ($T_C$) and helper T cells ($T_H$). $T_C$ cells directly lyse cells that are infected with viruses or certain parasites and also will secrete cytokines such as gamma-interferon in order to eradicate intracellular pathogens and tumors.

Virtually all cell types can serve as antigen-presenting cells for $T_C$ cells as long as they express MHC Class I molecules. In general, $T_C$ cells require antigen-presenting cells that are actively biosynthesizing antigen. During processing, the antigen is bound to a nascent Class I molecule in the ER and transported to the plasma membrane via the Golgi complex and post-Golgi complex vesicles. At the plasma membrane, the processed antigen sits in the groove of the MHC Class I molecule, where the processed antigen is available for binding to cell surface receptors of $T_C$ cells. Activation of $T_C$ cells requires interaction between multiple $T_C$ cell surface molecules and their respective ligands on antigen-presenting cells. Once activation has taken place, the lysing and cytokine secretion activity described above can begin.

Antigen processing is the structural modification and trafficking, within the proper subcellular compartments, of protein antigens that enable the determinants recognized by $T_C$ cells to interact with MHC molecules. As noted above, most, and possibly all, somatic cells expressing MHC Class I molecules constitutively process antigens and transport determinants to the cell surface for $T_C$ cell recognition. Antigen processing is thus required for the presentation of intact, folded proteins to $T_C$ cells. Commonly, antigen processing entails the generation of short peptides by cellular proteases, although some intact proteins productively associate with MHC molecules, indicating that proteolysis is not necessarily a component of antigen processing.

Two distinct pathways are used by cells to process antigens. The endosomal pathway is so named because it is accessed through the endosomal compartment. Determinants produced by this pathway usually associate with Class II MHC molecules. The other pathway is the cytosolic pathway. The cytosolic pathway is so named because it can be accessed from the cytosol of the cell by the synthesis of proteins within the cell, or by penetration of plasma or endosomal membranes by extracellular proteins. Such penetration may occur naturally through the fusion of the cell's membrane with a virus, or artificially by osmotic lysis of antigen-containing pinosomes. Determinants produced by cytosolic processing typically associate with Class I MHC molecules. The cytosolic pathway is able to process many different types of foreign proteins for presentation to $T_C$ cells.

Class I MHC molecules associate with antigens in a compartment of the ER. In this regard, it is important to note that the compound Brefeldin A acts by interfering with the normal vesicular traffic between the ER and the Golgi apparatus, and thus also has the effect of blocking the presentation of cytosolically processed antigen on the surface of what would otherwise be an antigen-presenting cell.

It can be seen from the above discussion that, in order to generate response by a cytotoxic T cell, it is generally necessary either to cause the target cell, which has been chosen as an antigen-presenting cell, to endogenously synthesize the protein antigen of interest, or to deliver exogenous protein antigen of interest directly into the cytosolic antigen processing pathway of the target cell. If the latter could be accomplished, a vaccine could be produced which would elicit cytotoxic T cells capable of killing virally or parasitically infected cells or tumor cells, thereby having particular usefulness for preventing three clinical types of diseases.

First, such vaccines could prevent infections caused by viruses such as papilloma or herpes virus which do not undergo a blood-borne phase of infection. This would be especially true in the case of human papilloma virus E7 protein, which is continuously cellularly expressed in the transformed phenotype, and would thus be particularly well suited to attack by sensitized cytotoxic T lymphocytes.

Secondly, there are those infections caused by viruses such as influenza or human immunodeficiency virus (HIV) or parasites whose outer proteins may have high antigenic variability making it difficult to design a vaccine capable of eliciting protective titers of high affinity antibodies with broad specificity. Certain viral internal proteins have less antigenic variation, and peptides derived from such proteins when associated with Class I MHC molecules, would render infected cells susceptible to lysis by sensitized cytotoxic T lymphocytes.

Thirdly, tumors and virally transformed cells express neoantigens that may be presented on Class I MHC molecules, thus rendering these cells suitable targets for cytotoxic T lymphocyte lysis.

Current vaccines generally focus on generating humoral (that is, antibody) responses of the immune system, rather than the cellular immune responses discussed above. Those that do generate cellular immune responses use attenuated live viruses which replicate intracellularly, introducing their constituents into an infected cell's antigen processing pathway as a result of being synthesized within the cell thereby being available for the appropriate protein processing pathway. Thus, there is a need for a non-replicating vaccine that will sensitize cytotoxic T lymphocytes to produce a cellular immune response with a significantly greater margin of safety.

The present invention meets this need by capitalizing on the ability of certain bacterial exotoxins to be internalized into cells through endocytosis via receptors on the cell surface and then translocate out of the resultant endosomes into the cellular compartment in which endogenous proteins are processed for presentation. These exotoxins have been hybridized with polypeptide or protein antigens, which are carried into the cytoplasm and are processed to peptides capable of association with Class I MHC molecules via the physiologic processes discussed above. Once associated with a Class I MHC molecule and presented on the surface of the antigen-presenting cell, they can sensitize cytotoxic T lymphocytes against other infected cells synthesizing the same polypeptide or protein. By virtue of these actions, the invention presents vaccines which can be effective in prophylaxis against viruses, parasites and malignancies.

It is an additional object of the present invention to produce hybrid proteins of certain bacterial exotoxins having translocation domains, hybridized with polypeptides or proteins selected for their antigenic activity, which hybrids will be useful as probes for studying the intracellular processing and subsequent presentation of endogenously synthesized cytoplasmic proteins.

## BRIEF DESCRIPTION OF THE DRAWINGS

Figure 1 shows the structural domains of Pseudomonas exotoxin, along with the numbers of the amino acid residues that define the known limits of the structural domains. Amino acid residues are numbered as defined in Gray, et al, PNAS USA 81 = 2645-2649(1984).

Figure 2 is a restriction map for plasmid pVC45-DF+T.

Figure 3 is a restriction map for plasmid pBluescript II SK.

Figure 4 is a restriction map for plasmid pBR322.

Figure 5 is a graph showing the results of using hybrid construct PEMa in immunologically sensitizing U-2 OS cells, a human cell line.

Figure 6 shows that a hybrid protein made of the binding and translocating domains of Pseudomonas exotoxin and a peptide epitope of influenza A matrix protein can competitively prevent the intact Pseudomonas exotoxin from binding to and killing target cells.

## SUMMARY OF THE INVENTION

The invention is the recombinant DNA sequences coding for a hybrid protein of two species, the first species being a modified bacterial toxin that has a translocating domain. The second species is a polypeptide or protein. The polypeptide or protein is exogenous to an antigen-presenting cell of interest. The hybrid of the bacterial toxin and the exogenous polypeptide or protein are constructed in such a way as to be capable of eliciting an immune response by cytotoxic T lymphocytes. Also included are suitable plasmids and methods of using the recombinant sequences to obtain the hybrid proteins of interest.

A preferred bacterial toxin is a modified Pseudomonas exotoxin. Pseudomonas exotoxin is known to consist of four structural domains, namely Ia, II, Ib and III. This is shown at Figure 1, along with the numbers of the amino acid residues that define the known limits of the structural domains. More preferably, the Pseudomonas exotoxin is modified by deletion of structural domain III, that is the ADP-ribosylating structural domain, although alternatively domain III need not be entirely deleted, but may rather be sufficiently altered in its amino acid sequence so as to render it enzymatically nonfunctional as an ADP-ribosylating enzyme. Most preferably, the modified bacterial toxin has only a cellular recognition domain and a translocating domain, (with or without the 5 C-terminal amino acids of Domain III added to the C-terminus of the polypeptide or protein antigen), or even just the translocating domain with or without targeting ligand. In the case of Pseudomonas exotoxin, the cellular recognition domain and translocating domain are known to exist within structural domains Ia, II and Ib. Also most preferably, modified Pseudomonas exotoxins are arranged on the amino-terminal side of the hybrid, while the exogenous polypeptide or protein is arranged on the carboxyl-terminal side of the hybrid.

The exogenous polypeptide or protein, which is exogenous to an antigen-presenting cell of interest, is preferably a polypeptide or protein of viral origin. More preferably, the viral polypeptide is a viral protein fragment, and most preferably is taken from the group comprising the matrix protein of influenza A virus; residues 57 to 68 of the matrix protein of influenza A virus (the matix epitope known to bind MHC HLA-A2); the nucleoprotein of influenza A virus; or the GAG protein of human immunodeficiency virus-1.

Functionally, the hybrid is capable of eliciting an immune response by cytotoxic T lymphocytes, by virtue of being at least partially presented on an antigen-presenting cell surface. More specifically, the hybrid functionally is capable of being internalized by an antigen-presenting cell and further capable of being processed, via the endogenous protein processing pathway, on its way to at least partial presentation on the surface of the antigen-presenting cell.

The hybrid proteins preferably will use polypeptide or protein antigens for use as a vaccine, and most preferably will use viral antigens. Most preferably, these viral antigens will be conserved viral proteins. The hybrids will be incorporated in an amount sufficient to elicit an immune response by cytotoxic T lymphocytes into vaccines further comprising pharmaceutically acceptable carriers. The vaccines will be sufficient to immunize a host against the diseases influenza, acquired immunodeficiency syndrome, human papilloma virus, cytomegalovirus, Epstein-Barr virus, Rota virus, and respiratory syncytial virus, tumors and parasites.

The present invention further relates to recombinant DNA segments containing nucleotide sequences coding for the fused proteins described above, as well as plasmids and transformants harboring such recombinant DNA segments, as well as methods of producing the hybrid proteins using such recombinant DNA segments and methods of administration of the hybrid proteins as vaccines to hosts.

## DETAILED DESCRIPTION OF THE INVENTION

The term "translocating domain" shall mean a sequence of amino acid residues sufficient to confer on a polypeptide or protein the ability to translocate across a cell membrane into a cellular compartment for processing endogenous proteins.

The term "exogenous to an antigen-presenting cell" shall mean polypeptides that are not encoded by the unmutated genome of a given antigen-presenting cell.

The term "antigen-presenting cell" shall refer to a variety of cell types which carry antigen in a form that can stimulate cytotoxic T lymphocytes to an immunologic response.

The term "immune response" shall mean those cytotoxic processes of cell lysis and cytokine release engaged in by cytotoxic T lymphocytes that have been stimulated by antigen presented by an antigen-presenting cell. This term shall also include the ability of a host's cytotoxic T lymphocytes to retain their cytotoxic response to subsequent exposure to the same antigen that will lead to more rapid elimination of the antigen than in a non-immune state.

The term "presented on an antigen-presenting cell surface" shall mean that process by which an antigen is seated within a ligand site of a major histocompatability complex Class I protein on the surface of an antigen-presenting cell.

The term "being internalized by an antigen-presenting cell" shall mean the process of endocytosis resulting in endosome formation.

The term "cellular recognition domain" shall mean a sequence of amino acid residues in a polypeptide sufficient to confer on that polypeptide the ability to recognize a receptor site on the surface of a target cell.

The term "ADP ribosylating domain" shall mean a sequence of amino acids sufficient to confer on a polypeptide the ability to modify elongation. factor II within a cell, and thereby severly impair the viability of the cell or kill it.

The term "vaccine" shall mean a pharmaceutically acceptable suspension of a given therapeutic entity administered for the prevention, amelioration or treatment of infectious diseases.

The term "conserved viral protein" shall mean those viral proteins that do not vary from strain to strain of a given species of virus, or to those viral proteins that are generally unlikely to undergo mutation as a function of time in a given strain.

The term "arranged on the amino terminal side of said hybrid" shall mean that a peptide sequence has been inserted at any point between the amino terminus of a hybrid and the hybrid's middle amino acid residue.

The term "arranged on the carboxy terminal side of said hybrid" shall mean that a peptide sequence has been inserted at any point between the carboxy terminus of a hybrid and the hybrid's middle amino acid residue.

The term "transformant" shall mean an independent, self-replicating DNA molecule, and shall include plasmids.

The hybrid proteins of the present invention are fusion protein constructs of a bacterial toxin having a translocating domain fused to a polypeptide or protein that has been selected for its antigenicity for a given disease, as well as for being exogenous to a targeted antigen-presenting cell. A preferred bacterial toxin is the Pseudomonas exotoxin. This exotoxin is known to comprise four structural domains, as shown in Figure 1. These domains are designated Ia, II, Ib and III. Structural domain Ia is known to be necessary for binding of the exotoxin to a receptor site on the surface of a target cell. Structural domain II is known to be necessary for translocation of the exotoxin across an internal membrane the targeted cell. Part of structural III are known to be an ADP ribosylating enzyme that bind to the protein Elongation Factor 2, which generally results in the death of the target cell.

In a preferred embodiment of the present invention, structural domain III (or all domain III except for the C-terminal amino acids) has been deleted from the Pseudomonas exotoxin molecule, and has been replaced with one of several polypeptides or proteins chosen for their ability to act as antigens and therefore be useful as vaccines. The antigens used for vaccines include antigens of viruses whose hosts are higher vertebrates, such as antigen of influenza A virus, human immunodeficiency virus-1, human papilloma virus, cytomegalovirus, Epstein-Barr virus, Rota virus, and respiratory syncytial virus. Other viruses include herpes viruses such as herpes simplex virus, varicella-zoster virus, adult T cell leukemia virus, hepatitis B virus, hepatitis A virus, parvoviruses, papovaviruses, adenoviruses, pox viruses, reoviruses, paramyxoviruses, rhabdoviruses, arena-viruses, and coronaviruses. Other disease states can have antigens designed for them and used in alternative embodiments of the present invention, including antigens with pathogenic protozoa, such as malaria antigen.

The fusion proteins of the present invention are preferably manufactured through expression of recombinant DNA sequences.

The DNAs used in the practice of the invention may be natural or synthetic. The recombinant DNA segments containing the nucleotide sequences coding for the embodiments of the present invention can be prepared by the following general processes:

(a) A desired truncated gene is cut out from a plasmid in which it has been cloned, or the gene can be chemically synthesized;

(b) An appropriate linker is added thereto as needed, followed by construction of a fused gene; and

(c) The resulting fused protein gene is ligated down stream from a suitable promoter in an expression vector.

Techniques for cleaving and ligating DNA as used in the invention are generally well known to those of ordinary skill in the art and are described in Molecular Cloning, A Laboratory Manual, (1989) Sambrook, J., et al., Cold Spring Harbor Laboratory Press.

As the promoter used in the present invention, any promoter is usable as long as the promoter is suitable for expression in the host used for the gene expression. The promoters can be prepared enzymatically from the corresponding genes, or can be chemically synthesized.

Conditions for usage of all restriction enzymes were in accordance with those of the manufacturer, including instructions as to buffers and temperatures. The enzymes were obtained from New England Biolabs, Bethesda Research Laboratories (BRL), Boehringer Mannheim and Promega.

Ligations of vector and insert DNA's were performed with T4 DNA ligase in 66mM Tris-HCl, 5mM $MgCl_2$, lmMDTE, lmMATP, pH 7.5 at 15°C for up to 24 hours. In general, 1 to 200 ng of vector and 3-5x excess of insert DNA were preferred.

Selection of E. coli containing recombinant plasmids involve streaking the bacteria onto appropriate antibiotic containing LB agar plates or culturing in shaker flasks in LB liquid (Tryptone 10g/L, yeast extract 5g/L, NaCl 10g/L, pH 7.4) containing the appropriate antibiotic for selection when required. Choice of antibiotic for selection is determined by the resistance markers present on a given plasmid or vector. Preferably, vectors are selected by ampicillin.

Culturing of E. coli involves growing in Erlenmeyer flasks in LB supplemented with the appropriate antibiotic for selection in an incubation shaker at 250-300 rpm and 37°C. Other temperature from 25°-37°C could be utilized. When cells are grown for protein production, they are induced at $A_{560}$=1 with IPTG to a final concentration of 0.4 mM. Other cell densities in log phase growth can alternatively be chosen for induction.

Harvesting involves recovery of E. coli cells by centrifugation. For protein production, cells are harvested 3 hours after induction though, other times of harvesting could be chosen.

In the present invention, any vector, such as a plasmid, may be used as long as it can be replicated in a procaryotic or eucaryotic cell as a host.

By using the vector containing the recombinant DNA thus constructed, the host cell is transformed via the introduction of the vector DNA.

The host cell of choice is BL21 (DE3) cells (E. coli), obtained from F. Wm. Studier, Brookhaven National Laboratories, Stony Brook, N.Y. Reference is also made to Wood, J. Mol. Biol., 16:118-133 (1966) U.S. Patent No. 4,952,496, and Studier, et al., J. Mol. Biol. 189:113-130 (1986). However, any strain of E. coli containing an IPTG inducible T7 polymerase gene would be suitable. For routine cloning, E. coli strain DH5α(BRL) can be used.

BL21(DE3) strain of E. coli was acquired under license from W. F. Studier. Reference is made to Studier, W. F. et. al., Methods in Enzymology, Vol. 185, Ch. 6, pp 60-89 (1990). This strain is unique to the extent that it contains an inducible T7 polymerase gene. The strain has no amino acid, sugar or vitamin markers, so it can grow on any rich or defined bacterial medium. It can be grown between 25°C and 37°C. It needs aeration, and it needs IPTG for induction of the T7 polymerase.

In the present invention, the fused proteins can be separated and purified by appropriate combinations of well-known separating and purifying methods. These methods include methods utilizing a solubility differential such as salt precipitation and solvent precipitation, methods mainly utilizing a difference in molecular weight such as dialysis, ultrafiltration, gel filtration and SDS-polyacrylamide gel electrophoresis, methods utilizing a difference in electric charge such as ion-exchange column chromatography, methods utilizing specific affinity such as affinity chromatography, methods utilizing a difference in hydrophobicity such as reverse-phase high pressure liquid chromatography, methods utilizing a difference in isoelectric point, such as isoelectrofusing electrophoresis, and methods using denaturation and reduction and renaturation and oxidation.

Preferred embodiments of the invention will now be described in detail in the following non-limiting examples. The most preferred embodiments of the invention are any or all of those specifically set forth in these examples. These examples are not, however, to be construed as forming the only genus that is considered as the invention, and any combination or sub-combination of the examples may themselves form a genus. These examples further illustrate details for the preparation of various embodiments of the present invention. Those skilled in the art will readily understand that known variations of the conditions and processes of the following preparative procedures can be used to prepare these embodiments.

## EXAMPLE 1

### BS-PEMI-2

A 1.3kb NruI/SacII fragment of plasmid pVC45-DF+T (Fig. 2) (obtained from Dr. Ira Pastan of the National Institute of Health) containing the domain I and II coding regions of Pseudomonas exotoxin (PE) (Sequence ID No. 1) was subcloned into pBluescript II SK (Stratagene, Fig. 3) restricted with HincII and SacII. The resulting construct is designated BS-PE. The influenza MI (MI) gene (Sequence ID No. 2 and 3) which codes for the matrix protein of influenza A virus was subcloned into BS-PE restricted with SacII and SacI by amplifying the MI gene from pApr701 (P. Palase, Mt. Sinai Medical Center, New York, N.Y. pApr 701 consists of the MI gene cloned into the ECORI site of pBR322, shown at Fig. 4. Reference is made to Young, J.F. et. al, Expression of Influenza Virus Genes; The Origin of Pandemic Influenza Virus; 1983) by polymerase chain reaction (PCR) (Gene Amp® PCR Reagent Kit; Perkin Elmer Cetus, Norwalk, Conn. 06859) with oligonucleotide primers which added a SacII site adjacent to MI codon number 2 (Sequence ID No. 4) and a SacI site 3′ of the MI termination codon (Sequence ID No. 5). This plasmid is designated BS-PEMI-1.

The truncated ompA leader coding sequence was removed from the 5′ end of the fusion gene by replacing the small XhoI/HindIII fragment of BS-PEMI-1 with the oligonucleotide sequence shown in Sequence ID No. 6. The resulting plasmid is named BS-PEMI-2 and encodes a fusion gene consisting of Pseudomonas exotoxin amino acids 2 through 414 joined to MI amino acids 2 to 252 (Sequence ID No. 7 and 8).

## EXAMPLE 2

### pVC-ompA-PEMI-2

pVC45DF+T vector was prepared by restriction digestion with HindIII and EcoRI, followed by gel purification.

The PEMI insert fragment was prepared by restriction digestion of BS-PEMI-1 with SacI, followed by T4 DNA polymerase treatment to remove the 3′ overhang. EcoRI linkers were added to the blunted SacI site, followed by restriction digestion with HindIII. The HindIII-EcoRI fragment was gel purified (Molecular Cloning Manual, Gene Clean Kit, Bio 101, Inc. P.O. Box 2284, La Jolla, CA 92038) and ligated into the prepared pVC45-DF+T vector. The resulting construct was named pVC-ompA-PEMI-2.

The ompA signal sequence was removed from the construct by restriction digestion of pVC-ompA-PEMI-2 with XbaI and HindIII. An oligonucleotide fragment containing the T7 promoter, ribosome binding site and initiation sequence was ligated into the vector whose base sequence is shown at Sequence ID No. 9. The resulting plasmid construct was named pVC-PEMI-2 and encodes a T7 polymerase-driven gene fusion consisting of PE amino acids 2 through 414 joined to influenza MI amino acids 2 through 252. The 5′ and 3′ ends of the coding region, as well as the PE to MI fusion site and cytotoxic T lymphocyte epitope coding sequences (Rotzschke, O. et. al., Nature 348, 252 (1990) were confirmed by DNA sequencing.

## EXAMPLE 3

BS-PEMa

The influenza Ma sequence (coding for residues 57-68 of the influenza matrix protein) was obtained by amplifying a portion of the influenza M1 gene in pApr701 by polymerase chain reaction (PCR) with oligonucleotide primers which added a SacII site adjacent to influenza MI codon No. 57 (Sequence ID No. 10) and a termination codon and a SacI site 3′ of the MI codon No. 68 (Sequence ID No. 11). This fragment was cut with SacII and SacI and subcloned into BS-PE digested with SacII and SacI. The resulting plasmid is named BS-PEMa-1 and was verified by sequencing through the junctions and the Ma sequence itself.

## EXAMPLE 4

Subcloning of PEMa from BS-PEMaI into PVC45DF+T

The PEMa insert (Sequence ID No. 12) was prepared by restricting BS-PEMa-1 with SacI and removing the 3′ overhang by treatment with T4 DNA polymerase, then restricting with ApaI and gel purifying.

pVC45DF + T was restricted with EcoRI and the 5′ overhang filled in with Klenow enzyme treatment (Molecular Cloning Manual, ibid.). It was subsequently restricted with ApaI and gel purified. The vector and fragment were ligated together, and the resulting construction was named pVC-ompA-PEMa-1. The construction was verified by sequencing across the junctions and through Ma.

The ompA leader sequence was removed from pVC-ompA-PEMa-1 by digestion with XbaI and HindIII. An oligonucleotide fragment containing the T7 promoter, ribosome binding site, initiation sequence and a build-back of the 5′ end of the PE coding region (Sequence ID No. 13) was ligated to the vector. The resulting construction was named pVC-PEMa-1 and encodes a T7 polymerase driven gene fusion consisting of PE amino acids 2 to 414 joined to influenza MI amino acids 57 to 68 (Ma) Sequence ID No. 14 and 15. The 5′ end of pVC-PEMa-1 was verified by sequencing through the oligonucleotide fragment.

## EXAMPLE 5

Construction of pVC-PEBT

A control plasmid was constructed which encodes a T7 polymerase driven gene fusion consisting of PE amino acids 2 to 414 followed by termination codons. pVC-PEMI-2 was digested with SacII and EcoRI to remove the MI sequence. The vector was gel purified and ligated to an oligonucleotide that builds back PE codon No. 414 followed by termination signals shown in Sequence ID No. 16. The resulting construction was named pVC-PEBT (Sequence ID No. 17 and 18) and was verified by sequencing across the junctions and the oligonucleotide addition.

## EXAMPLE 6

BSK-PEMI

BSK-PEMI was made from BS-PEMI by the replacement of the 21 base pair XhoI/HindIII fragment with a 24 base pair fragment encoding a consensus eucaryotic ribosome binding site (Sequence ID No. 19). The purpose of the construct was to increase the yields of in vitro translated PEMI protein. Thus, an additional object of the invention is to increase yields of translated PEMI protein.

EXAMPLE 7

pVCPE/2 (pVC45DF+T/2)

pVCPE/2 was made by replacing the 105 base pair PpuMI/EcoRI fragment of pVC45DF+T with a 46 base pair DNA fragment encoding an inframe duplication of PE codons 604 to 613 flanked by unique cloning sites (Sequence ID No. 20). This construct is used for generating full-length molecules of PE with the deletion of residue 553 resulting in an inactivated toxin domain (sequence ID No. 21 and 22) fused to protein segments of choice between PE codons 604 and 605. One may replace the ompA signal sequence with the promoter/ribosome binding site as described for PVC-PEMI-2.

EXAMPLE 8

pVCPE/2-Ma

pVCPE/2-Ma was made by ligating into the XmaI site of pVCPE/2 a 48 base pair DNA fragment encoding amino acids 55 through 67 (Sequence ID No. 23). This construct expresses in E. coli full-length PE with MI amino acids 55 through 67 inserted between PE amino acid 604 and 605 (Sequence ID No. 24 and 25). One may replace the ompA signal sequence with the promoter/ribosome binding site as described for pVC-PEMI-2.

EXAMPLE 9

pVCPE/2-MI:15-106

pVCPE/2-MI:15-106 was made by subcloning a PCR-amplified DNA fragment encoding MI amino acids 15 through 106 into the XmaI site of pVCPE/2. The sequence of the oligonucleotide primers used to amplify the MI segment are those shown at Sequence ID No. 26 and 27, respectively. This construct expresses in E. coli full length PE with MI amino acids 15 through 106 inserted between PE amino acid 604 and 605 (Sequence ID No. 28 and 29). One may replace the ompA signal sequence with the promoter/ribosome binding site as described for pVC-PEMI-2.

EXAMPLE 10

pVCPEdel(403-613)

pVCPEdel(403-613) was made by restricting pVC45DF+T with SacII followed by elimination of the 3′ SacII overhang with T4 DNA polymerase and the ligation of a 3-frame termination linker whose nucleic acid sequence is given at Sequence ID No. 30. This construct will express PE domains I, II and Ib only, fused to the ompA leader in E. coli.

EXAMPLE 11

pVCPEdel(403-505)

pVCPEdel(403-505) was made by restricting pVC45DF+T with SacII and XhoI followed by removal of restriction overhangs with mung bean nuclease (New England Biolabs). The vector fragment was recovered and reclosed with DNA lipase. This construct will express in E. coli the PE protein lacking amino acids 403 through 505.

EXAMPLE 12

pVCPEdel(494-505)

pVCPEdel(494-505) was made by restricting pVC45DF+T with BamHI and XhoI followed by the filling in of the 5′ overhangs with Klenow fragment. The vector fragment was recovered and reclosed with DNA ligase. This construct will express in E. coli the PE protein lacking amino acids 494 through 505.

EXAMPLE 13

pVCPEdel(494-610)

pVCPEdel(494-610) was made by restricting PVC45DF+T with BamHI and PpuMI followed by the filling in of the 5′ overhangs with Klenow fragment. The vector fragment was recovered and reclosed with DNA ligase. This construct will express in E. coli the PE protein lacking amino acids 494 through 610. All of the pVCPEdel plasmids were useful in determining to what extent the toxin domain of PE could be truncated without resulting in the expression of an insoluble protein in E. coli. It thus became an additional object of the invention to provide hybrids having the minimal toxin domain of PE that would retain water solubility.

EXAMPLE 14

Addition of Sequences Between pE and MI in pVC-PEMI-2

Oligonucleotide linkers can be added at the SacII site between PE and MI in pVC-PEM-2. These linkers can be designed to add cleavage sites and/or signal sequences which can help the MI portion of the fusion protein to become available for presentation within the cell. SacII digestion cleaves the gene between the last two PE codons (for amino acids 413 and 414) and provides an appropriate site for such additions.

The following four constructions have been made by inserting linkers at the SacII site. The constructions have been verified by sequencing across the SacII junctions and through the complete linker.

EXAMPLE 15

pVC-PE-RK-MI

This vector contains an ARG LYS(RK) cleavage site inserted into the SacII site, using an oligonucleotide linker as shown in Sequence ID No 31. The resulting amino acid sequence between amino acids 413 and 414 of PE is Gly Gly Arg Lys Ser.

EXAMPLE 16

pVC-PE-RKSigI-MI

This vector contains an ARG LYS(RK) cleavage site and the signal sequence that is shown in Sequence ID No. 32 from the Influenza A hemagglutinin (HA) protein inserted at the SacII site, using the oligonucleotide linker disclosed at Sequence ID No. 33. The resulting amino acid sequence between amino acids 413 and 414 of PE is also as shown in Sequence ID No. 34.

EXAMPLE 17

PVC-PE-Sig1-MI

This vector contains the signal sequence of HA without the RK cleavage site inserted into the SacII site using the oligonucleotide linker shown at Sequence ID No. 35. The resulting amino acid sequence between amino acids 413 and 414 of PE is also as shown at Sequence ID No. 36.

EXAMPLE 18

pVC-PE-Sig2-MI

This vector contains the signal sequence shown at Sequence ID No. 37, derived from amino acids 22 to 48 from ovalbumin inserted into the SacII site, using the oligonucleotide linker of Sequence ID No. 38. The resulting amino acid sequence between amino acids 413 and 414 of PE is also as that shown in Sequence ID No. 39.

Addition of Sequences Between

PE and Ma In pVC-PEMa-1

Oligonucleotide linkers can be added at the SacII site between PE and Ma in pVC-PEMa-1. These linkers can be designed to add cleavage sites and/or signal sequences which can help the Ma peptide to become available for presentation within the cell. SacII digestion cleaves the gene between the last two PE codons (for amino acids 413 and 414) and thus provides an appropriate site for such additions.

The following four examples have been made by inserting linkers at the SacII site. The constructions have been verified by sequencing across the SacII junctions and through the complete linker.

EXAMPLE 19

pVC-PE-RKSig1-Ma

This vector contains an ARG LYS (RK) cleavage site and the signal sequence from the Influenza A hemagglutimin (HA) protein inserted into a blunted SacII site, using the oligonucleotide linker shown at Sequence ID No. 40. The resulting amino acid sequence between amino acids 413 and 414 of PE exotoxin is also as shown at Sequence ID No. 41.

EXAMPLE 20

pVC-PE-Sig1-Ma

This vector contains the single sequence of HA without a cleavage site inserted into a blunted SacII site using the oligonucleotide linkers shown in Sequence ID No. 42. The resulting amino acid sequence between amino acids 413 and 414 of PE is also as shown in Sequence ID No. 43.

EXAMPLE 21

pVC-PE-Sig2-Ma

This vector contains a signal sequence derived from amino acids 22 through 48 from ovalbumin inserted into a blunted SacII site, using the oligonucleotide linker as seen in Sequence ID No. 44. The resulting amino acid sequence between amino acids 413 and 414 of PE is also as shown in Sequence ID No. 45.

EXAMPLE 22

pVC-PE-Sig1Sig2-MA

This vector contains the signal sequence derived from HA, followed by the signal sequence from ovalbumin inserted into the SacII site, using the oligonucleotide linker shown at Sequence ID No. 46. The resulting amino acid sequence between amino acids 413 and 414 of PE is also as shown at Sequence ID No. 47.

EXAMPLE 23

BSPEMIc5aa

The plasmid BSPEMI-2 was digested with SacI and StuI and ligated to the oligonucleotide linker shown at Sequence No. 48. This linker builds back the C-terminus of the MI protein and adds the last five amino acids from the C-terminus of the PE protein, whose sequence is Arg Glu Asp Leu Lys, followed by a termination codon. This also incorporates an EcoRI site. The resulting plasmid was named BSPEMIc5aa and was sequenced across the junctions (Sequence ID No. 49 and 50) and the linker for verification of the construction.

EXAMPLE 24

pVC-PEMIc5aa

The plasmid BSPEMIc5aa was digested with HindIII and EcoRI and 1.8 kb PEMIc5aa fragment was gel purified. The plasmid pVC-PEMI-2 was digested with HindIII and EcoRI and the 3.2 kb vector fragment was ligated to the 1.8 kb PEMIc5aa fragment and the resulting plasmid was named pVC-PEMIc5aa. The 5′ and 3′ ends of the PEMIc5aa insert were verified by sequencing.

EXAMPLE 25

pVC-PENPc5aa

A fragment containing the nucleoprotein (NP) of Influenza A virus was obtained from plasmid pApr501 (obtained from Peter Palase, Mt. Sinai Medical Center, New York, N.Y. pApr501 is said nucleoprotein gene cloned into the EcoRI site of pBR322, (Fig. 4) by polymerase chain reaction with oligonucleotide primers which added a SacII site adjacent to the ATG codon of NP to give the sequence shown at Sequence ID No. 51, and the last 5 amino acids of PE followed by a termination codon and an EcoRI site to the 3′ end of NP to give the sequence shown at Sequence ID No. 52. The polymerase chain reaction fragment was digested with SacII and EcoRI and ligated to the plasmid pVC-PEMI-2 digested with SacII and EcoRI. The resulting plasmid is named pVC-PENPc5aa. The 5′ and 3′ ends of the PENPc5aa insert (Sequence ID No. 53 and 54) were verified by sequencing. This construction fuses the binding and translocation domains of PE to the Influenza A nucleoprotein.

EXAMPLE 26

pVC-ompA-PEGAG

The HIV GAG gene was obtained from plasmid HIVpBR322 (obtained from Ron Diehl Merck, Sharpe and Dohme Research Laboratories, West Point, PA., Fig. 5) by polymerase chain reaction with oligonucleotides that added a SacII site adjacent to the ATG codon of GAG to give the nucleotide sequence shown at Sequence ID No. 55, and a SacI site immediately after the termination codon at the 3′ end to give the nucleotide sequence at Sequence ID No. 56. The polymerase chain reaction fragment was digested with SacII and ligated to plasmid pVC45DF+T, which had been digested with EcoRI, the 5′ overhang filled in by Klenow fragment, and digested with SacII. The resulting plasmid was named pVC-ompA-PEGAG (Sequence ID No. 57 and 58) and was verified by a partial sequence at the SacII junction.
This construction fused the binding and translocation domains of PE to the GAG gene of HIV-1 virus. The fusion protein contains an ompA leader sequence. Alternatively, any vector containing the complete coding region for HIV GAG can be used with these oligomers to generate the HIV GAG gene by PCR.

EXAMPLE 27

Expression of PEMI, PEMa and PEBT

Frozen competent BL21(DE3) cells (as described by Studier, et al. Mol. Biol., 189, 113-130, 1986) were prepared as described (DNA cloning, Vol. 1, p. 121, Ed. D N Glover, IRL Press, Wash., D.C.).

BL21(DE3) cells were transformed with pVC-PEMI-2, pVC-PEMa-1, or pVC-PEBT as described below (this can be performed with pVC-PE fusion plasmids in general) and transformants were selected on L-Amp plates. Fresh transformants were used to inoculate L-Amp liquid cultures at A560=0.1. Cultures were grown at 37°C with vigorous aeration and induced at A560=1.0 with IPTG to a final concentration of 0.4 mM. Cultures were harvested after 3 hours of induction and the cell pellets used for protein extraction and purification (Protein Structure: A Practical Approach, T.E. Creighton, ed., IRL Press at Oxford Univ. Press, Ch. 9, 191 (1989)).

Transformation Procedure

A bath of dry ice/ethanol was prepared and maintained at -70°C. Competent cells were removed from a -70°C freezer and thawed on ice. A sufficient number of 17 x 100 mm polypropylene tubes (Falcon 2059) were placed on ice. 100 μl aliquots of gently mixed cells were prepared in the chilled polypropylene tubes. DNA was added by moving a pipette through the cells while dispensing; the cells were then gently shaken for 5 seconds after addition. The cells were incubated on ice for 30 minutes, then heat-shocked in a 42°C water bath for 45 seconds without shaking. The cells were again placed on ice for 2 minutes. 0.9 ml of S.O.C. reagent (Bactotryptone 2%, Yeast Extract 0.5%, NaCl 10mM, KCl 2.5mM, $MgCl_2 \cdot MgSO_4$ 20mM, Glucose 20mM and distilled water, up to 100 ml) was added and the mixture shaken for 1 hour at 225 rpm and 37°C, then plated on antibiotic plates, spread gently.

EXAMPLE 28

Incubation of U-2 OS Cells With $^{51}Cr$ and Protein/PEMa

U-2 OS cells (ATCC) were harvested from flasks, after a 1X wash with RCM 8, using 1mM EDTA. The flasks were incubated at 37°C for 10 minutes. until cells were nonadherent. Five ml. of U-2 OS medium [McCoy's 5A (GIBCO) supplemented with 15% fetal bovine serum (HyClone) and penicillin 100 U/ml and streptomycin 100 µg/ml (GIBCO)] was added, and the cells were centrifuged for 10 minutes at 210 x g.

Cells were resuspended in U-2 OS medium at 8.5 x $10_5$/ml. To each well of a 12-well plate, 0.7 ml of cell suspension was added. Negative controls include U-2 OS medium alone and PEBT. The positive control for sensitization of U-2 OS cells is KKAMI (2 µg/ml), from M. Gammon and H. Zweerink (Merck, Sharp and Dohme Research Laboratories, Rahway, NJ). PEMa was added at 0.2µM or greater well concentration. Simultaneously, 137.5 µCi of $^{51}Cr$ (Amersham) was added to each well. Medium was added to all wells to bring the total volume to 1 ml. This was placed at 37°C, 5.5% $CO_2$ for 14 hours.

EXAMPLE 29

Assay Protocol for CTL Activity Against Sensitized U-2 OS Targets

After the 14 hour incubation, U-2 OS were removed, after a 1X RCM 8 wash using 1mM EDTA. Plates were incubated at 37°C for 10 minutes until cells were nonadherent. K medium [RPMI 1640 (GIBCO) supplemented with 10% fetal bovine serum (HyClone), 10 mM HEPES (GIBCO), 2 mM L-glutamine (GIBCO), penicillin 100 U/ml and streptomycin 100 µg/ml (GIBCO), and 50 µm 2-mercaptoethanol (Bio-Rad)] was added to give a total volume of 10 ml; cells were centrifuged for 10 minutes at 210 x g. The cells were incubated at room temperature for 10 minutes in 10 ml of K medium before entering the second centrifugation. The cells were then resuspended in 1 ml of K medium, counted, and resuspended to 1 x $10^5$/ml in K medium.

Human cytotoxic T lymphocytes, generated from one donor, were harvested, centrifuged for 10 minutes at 92 x g, and resuspended in K medium at 2.5 x $10^6$/ml.

100 µl of human CTLs were added to each well of a 96-well U-bottom microtiter plate (CoStar). 100 µl of the U-2 OS $^{51}Cr$-labeled targets were also added to these wells for a final effector/target ratio of 25:1. Spontaneous $^{51}Cr$ release was determined by incubating U-2 OS cells with 100 µl of K medium alone. The maximal release was determined by adding 100 µl of 6 M HCl to 100 µl of targets. The plates were quickly centrifuged to bring down the cells, and incubated for 2 hours at 37°C.

After this 2 hour incubation, the plates were centrifuged for 5 minutes, 330 x g, 5°C; 30 µl of supernatant was harvested from each well onto a plastic-backed filtermat (Pharmacia/LKB). The mat was dried in the microwave for 3 minutes. on medium-high power. The mat was placed into a sample bag with 10 ml of BetaPlate Scint, heat sealed and placed into the BetaPlate 1205 counter (Pharmacia/LKB). Results were expressed as % specific lysis, defined as:

$$\%\text{ specific lysis} = \frac{\text{Experimental} - \text{Spontaneous}}{\text{Maximal} - \text{Spontaneous}} \times 100$$

where

Experimental = counts per minute from the 30 µl of supernatant harvested from the wells containing targets plus human cytotoxic T lymphocytes, as determined by a BetaPlate 1205 counter;
Spontaneous = counts per minute from the 30 µl of supernatant harvested from the wells containing targets plus medium alone, as determined by the BetaPlate 1205 counter; and
Maximal = counts per minute from the 30 µl of supernatant harvested from the wells containing target plus 6M HCl (Fisher Scientific), as determined by the BetaPlate 1205 counter.

Results are presented graphically in Fig. 5, with U-2 OS medium alone and PEBT as negative controls, and KKAMI as a positive control. Greater that 10% specific lysis is considered a positive response (Cerottini, et.al., J. Exp. Med. 140:703, 1974).

EXAMPLE 30

Generation of MI-specific Human Cytotoxic T Lymphocytes

Original stock of human cytotoxic T lymphocytes was derived by harvesting blood from one donor into a syringe (Becton Dickinson) containing 25 U of heparin for each ml of whole blood (Elkins-Sinn, Inc.). The heparinized blood was pipetted directly into a Leucoprep tube (Becton Dickinson) and centrifuged for 20 minutes at 1700 X g. The buffy coat which was seen just above the interface was removed, centrifuged for 10 minutes at 92 X g, and washed twice in RPMI 1640 (GIBCO). The peripheral blood mononuclear cells (PBLs) recovered from the Leucoprep procedure were resuspended in 10 ml of CTL medium [RPMI 1640 (GIBCO) supplemented with 10% donor or pooled human plasma, 4 mM L-glutamine, 10 mM HEPES, penicillin 100 U/ml and streptomycin 100 μg/ml (GIBCO)] at 1 X $10^6$/ml.

MI peptide (received from M. Gammon and H. Zweerink, MSDRL, Rahway; 2 mg/ml stock) in DMSO was diluted 1:10 in RPMI 1640 (GIBCO). MI peptide was added to the 10 ml of lymphocytes at a final concentration of 5 μg/ml. The cells were then plated at 1.5 X $10^6$/well in 24-well plates (Nunc).

Two U/ml of Interleukin-2 ala-125 (Amgen) was added on Day 3. The cell density was adjusted to 1 X $10^6$/ml as needed, and the medium was supplemented with 2 U/ml additional Interleukin-2 to compensate for the increase in volume. Cells were restimulated with peptide-pulsed peripheral blood lymphocytes every 7 days as described below. Interleukin-2 ala-125 (Amgen) was replenished every 3 days.

Cytotoxic T lymphocytes and unstimulated PBLs were frozen (CryoMed) in a mixture of 70% RPMI 1640 (GIBCO), 20% fetal bovine serum (HyClone), and 10% dimethyl sulfoxide (Sigma) and thawed as needed.

EXAMPLE 31

Recovery and Restimulation of Frozen CTL's

Cytotoxic T lymphocytes (CTL's) were thawed in a 37° water bath and then resuspended in 35 ml of CTL medium [RPMI 1640 (GIBCO) supplemented with 10% donor or pooled human plasma, 4 mM L-glutamine, 10 mM HEPES, penicillin 100 U/ml and streptomycin 100 μg/ml (GIBCO]. The cytotoxic T lymphocytes were then placed at 37°, 5% $CO_2$ for 1 hour. The cell suspension was centrifuged for 10 minutes at 92 X g. The cells were resuspended at 5 X $10^5$/ml in CTL medium.

The source of stimulator cells for the freshly thawed cytotoxic T lymphocytes was freshly harvested PBL, which had been collected using the Leucoprep method described above. For peptide pulsing, an appropriate number (2 x $10^6$ - $10^7$) of PBL were centrifuged, the supernatant was aspirated, and KKAMI at 200 μg/ml in RPMI 1640 (GIBCO) plus 10% DMSO (Sigma) was added at the rate of 100 μl of KKAMI for every $10^7$ cells. The cells were incubated for 1 hour at 37°, 5% $CO_2$. The peptide-pulsed peripheral blood lymphocytes were irradiated with 2,000 Rads using a $^{60}Co$ source. The cells were washed once in RPMI 1640, centrifuged for 10 minutes at 92 X g, and resuspended in CTL medium at 1 X $10^6$/ml.

Equal volumes of cytotoxic T lymphocytes and irradiated, peptide-pulsed peripheral blood lympocytes were mixed together for a final ratio of 1 CTL:2 peptide-pulsed PBL. Interleukin-2 ala-125 (Amgen) was added at a final concentration of 2 U/ml. The cells were thoroughly mixed together with the Interleukin-2 ala-125 and 1.2 ml was plated into each well of a 48-well plate (CoStar).

The cells were counted and Interleukin-2 ala-125 was replenished every 3 days. This was achieved by pooling all the wells into a centrifuge tube, counting the cells in a hemocytometer counting chamber, adjusting the cells to 1 X $10^6$/ml with CTL medium, and adding 2 U/ml of Interleukin-2 ala-125. Then 1.5 X $10^6$ cytotoxic T lymphocytes in 1.5 ml of CTL medium with Interleukin-2 ala-125 were plated into each well of a 24-well plate (CoStar). the restimulation process was repeated every seven days, at which time frozen PBL's were then used as the source of stimulators.

Example 32

Binding of PEMa to the PE receptor

PEMa was used in a binding/competition assay to compete with PE for the PE receptor on U-2 OS cells. In doing so, PEMa was shown in Figure 6 to protect the cells from the toxic effects of PE. Therefore, replacement of the toxin domain of PE with the Influenza matrix peptide (amino acids 57-68) did not prohibit the binding of this chimeric protein to the PE receptor. This suggests that the ability of PEMa to sensitize target cells for lysis by CTLs specific for the matrix peptide is mediated through PE receptor-mediated uptake and processing.

U-2 cells were grown to a density of 20,000 cells/100µl in 960 well plates. Cells were preincubated with PEMA (0,0.1, 1, 10 and 50 µg in 100 µl of complete McCoy's 5A medium) for 30 minutes at 37°C, followed by incubation with or without PE(10 ng) for 2 minutes. This represents a 0-, 10-, 100-, 1000-, and 5000-fold excess of PEMA over PE, respectively. Cells were washed with McCoy's medium (3 x 200 µl); then incubated with [$^{35}$S]methionine (2 µCi/100 µl) for an additional 5 hours at 37°C and washed (3 x 200 µl). Cells were lysed in 10mM EDTA (100 µl) and aliquots (5 µl) were spotted onto Whatman 3MM filters. Incorporation of radioactivity was assayed by TCA precipitation of the cellular proteins onto the filter papers by immersion into ice-cold TCA (10% w/v) for at least 1 hour. Filters were washed once with 5% TCA and 3 times with ethanol and dried. Radioactivity was determined by liquid scintillation counting. Incorporation of [$^{35}$S]methionine into the TCA-precipitable pool of cellular proteins in the absence (open circles) or presence (closed circles) of PE is shown as a function of lop excess PEMa. Error bars represent +/-SEM for n=9. Using a one-tailed t-test, incorporation of [$^{35}$S]methionine was determined to be significantly lower in the presence of PE than in the absence of PE at 0-, 10-, and 100-fold excesses of PEMa (99.5%, 99.5% and 95% confidence limits, respectively). However, at 1000- and 5000-fold excesses of PEMa, incorporation was not significantly different in the presence or absence of PE.

Following preparation of the protein hybrids of the present invention, a suspension of the protein-hybrids suitable for injection into the host animal must be prepared. Typical suspension vehicles include sterile saline and sterile water for injection. Various agents may be added as preservatives including benzethonium chloride (0.0025%), phenol (0.5%), thiomersal (1:10,000). Strength of the vaccine will be measured as mass of fusion protein which generates a protective response, defined by in vitro/in vivo results, per given host species, a method known to those of ordinary skill in the art.

The suspensions for injection must, of course, be prepared under sterile conditions, in which there is a total absence of living organisms and absolute freedom from biological contamination present in the suspension for injection.

Although water is always the solvent of choice for an injectable preparation, co-solvents that may be additionally present include ethyl alcohol, glycerin, propylene glycol, polyethylene glycol and dimethylacetamide. Buffers may be added, including acidic acid, citric acid or phosphoric acid systems. Antioxidants can include ascorbic acid, BHA, BHT, sodium bisulfite, and sodium metabisulfite. Tonicity can be adjusted with agents such as dextrose, sodium chloride and sodium sulfate.

Aseptic manufacture of vaccines, including their packaging, is conducted according to methods well known to those of ordinary skill in the art, and as described in standard texts on the subject, including Lachman, L., et al., The Theory And Practice of Industrial Pharmacy, Dittert, L., ed, Sprowl's American Pharmacy; and Remington's Pharmaceutical Sciences.

While the invention has been described and illustrated in reference to certain preferred embodiments thereof, those skilled in the art will appreciate that various changes, modifications and substitutions can be made therein without departing from the spirit and scope of the invention. It is intended, therefore, that the invention be limited only by the scope of the claims which follow, and that such claims be interpreted as broadly as is reasonable.

SEQUENCE LISTING

(2) INFORMATION FOR SEQ ID NO:1:

(i) SEQUENCE CHARACTERISTICS:
(A) LENGTH: 1294 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

```
TCGCGATTGC AGTGGCACTG GCTGGTTTCG CTACCGTAGC GCAGGCCGCG AATTTGGCCG      60

AAGAAGCTTT CGACCTCTGG AACGAATGCG CCAAAGCCTG CGTGCTCGAC CTCAAGGACG     120

GCGTGCGTTC CAGCCGCATG AGCGTCGACC CGGCCATCGC CGACACCAAC GGCCAGGGCG     180

TGCTGCACTA CTCCATGGTC CTGGAGGGCG GCAACGACGC GCTCAAGCTG GCCATCGACA     240

ACGCCCTCAG CATCACCAGC GACGGCCTGA CCATCCGCCT CGAAGGCGGC GTCGAGCCGA     300

ACAAGCCGGT GCGCTACAGC TACACGCGCC AGGCGCGCGG CAGTTGGTCG CTGAACTGGC     360

TGGTACCGAT CGGCCACGAG AAGCCCTCGA ACATCAAGGT GTTCATCCAC GAACTGAACG     420

CCGGCAACCA GCTCAGCCAC ATGTCGCCGA TCTACACCAT CGAGATGGGC GACGAGTTGC     480

TGGCGAAGCT GGCGCGCGAT GCCACCTTCT TCGTCAGGGC GCACGAGAGC AACGAGATGC     540

AGCCGACGCT CGCCATCAGC CATGCCGGGG TCAGCGTGGT CATGGCCCAG ACCCAGCCGC     600

GCCGGGAAAA GCGCTGGAGC GAATGGGCCA GCGGCAAGGT GTTGTGCCTG CTCGACCCGC     660

TGGACGGGGT CTACAACTAC CTCGCCCAGC AACGCTGCAA CCTCGACGAT ACCTGGGAAG     720

GCAAGATCTA CCGGGTGCTC GCCGGCAACC CGGCGAAGCA TGACCTGGAC ATCAAACCCA     780

CGGTCATCAG TCATCGCCTG CACTTTCCCG AGGGCGGCAG CCTGGCCGCG CTGACCGCGC     840

ACCAGGCTTG CCACCTGCCG CTGGAGACTT TCACCCGTCA TCGCCAGCCG CGCGGCTGGG     900

AACAACTGGA GCAGTGCGGC TATCCGGTGC AGCGGCTGGT CGCCCTCTAC CTGGCGGCGC     960

GGCTGTCGTG GAACCAGGTC GACCAGGTGA TCCGCAACGC CCTGGCCAGC CCCGGCAGCG    1020
```

GCGGCGACCT GGGCGAAGCG ATCCGCGAGC AGCCGGAGCA GGCCCGTCTG GCCCTGACCC 1080

TGGCCGCCGC CGAGAGCGAG CGCTTCGTCC GGCAGGGCAC CGGCAACGAC GAGGCCGGCG 1140

CGGCCAACGC CGACGTGGTG AGCCTGACCT GCCCGGTCGC CGCCGGTGAA TGCGCGGGCC 1200

CGGCGGACAG CGGCGACGCC CTGCTGGAGC GCAACTATCC CACTGGCGCG GAGTTCCTCG 1260

GCGACGGCGG CGACGTCAGC TTCAGCACCC GCGG 1294

(2) INFORMATION FOR SEQ ID NO:2:

(i) SEQUENCE CHARACTERISTICS:
(A) LENGTH: 759 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:2:

ATGAGTCTTC TAACCGAGGT CGAAACGTAC GTTCTCTCTA TCATCCCGTC AGGCCCCCTC 60

AAAGCCGAGA TCGCACAGAG ACTTGAAGAT GTCTTTGCAG GGAAGAACAC CGATCTTGAG 120

GTTCTCATGG AATGGCTAAA GACAAGACCA ATCCTGTCAC CTCTGACTAA GGGGATTTTA 180

GGATTTGTGT TCACGCTCAC CGTGCCCAGT GAGCGAGGAC TGCAGCGTAG ACGCTTTGTC 240

CAAAATGCCC TTAATGGGAA CGGGGATCCA AATAACATGG ACAAAGCAGT TAAACTGTAT 300

AGGAAGCTCA AGAGGGAGAT AACATTCCAT GGGGCCAAAG AAATCTCACT CAGTTATTCT 360

GCTGGTGCAC TTGCCAGTTG TATGGGCCTC ATATACAACA GGATGGGGGC TGTGACCACT 420

GAAGTGGCAT TTGGCCTGGT ATGTGCAACC TGTGAACAGA TTGCTGACTC CCAGCATCGG 480

TCTCATAGGC AAATGGTGAC AACAACCAAC CCACTAATCA GACATGAGAA CAGAATGGTT 540

TTAGCCAGCA CTACAGCTAA GGCTATGGAG CAAATGGCTG GATCGAGTGA GCAAGCAGCA 600

GAGGCCATGG AGGTTGCTAG TCAGGCTAGG CAAATGGTGC AAGCGATGAG AACCATTGGG 660

ACTCATCCTA GCTCCAGTGC TGGTCTGAAA AATGATCTTC TTGAAAATTT GCAGGCCTAT 720

CAGAAACGAA TGGGGGTGCA GATGCAACGG TTCAAGTGA 759

(2) INFORMATION FOR SEQ ID NO:3:

(i) SEQUENCE CHARACTERISTICS:
(A) LENGTH: 253 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:3:

Met Ser Leu Leu Thr Glu Val Glu Thr Tyr Val Leu Ser Ile Ile Pro
1 5 10 15

Ser Gly Pro Leu Lys Ala Glu Ile Ala Gln Arg Leu Glu Asp Val Phe
20 25 30

Ala Gly Lys Asn Thr Asp Leu Glu Val Leu Met Glu Trp Leu Lys Thr
35 40 45

Arg Pro Ile Leu Ser Pro Leu Thr Lys Gly Ile Leu Gly Phe Val Phe
50 55 60

Thr Leu Thr Val Pro Ser Glu Arg Gly Leu Gln Arg Arg Arg Phe Val
65 70 75 80

Gln Asn Ala Leu Asn Gly Asn Gly Asp Pro Asn Asn Met Asp Lys Ala
85 90 95

Val Lys Leu Tyr Arg Lys Leu Lys Arg Glu Ile Thr Phe His Gly Ala
100 105 110

Lys Glu Ile Ser Leu Ser Tyr Ser Ala Gly Ala Leu Ala Ser Cys Met
115 120 125

Gly Leu Ile Tyr Asn Arg Met Gly Ala Val Thr Thr Glu Val Ala Phe
130 135 140

Gly Leu Val Cys Ala Thr Cys Glu Gln Ile Ala Asp Ser Gln His Arg
145 150 155 160

```
Ser His Arg Gln Met Val Thr Thr Thr Asn Pro Leu Ile Arg His Glu
                165                 170                 175

Asn Arg Met Val Leu Ala Ser Thr Thr Ala Lys Ala Met Glu Gln Met
            180                 185                 190

Ala Gly Ser Ser Glu Gln Ala Ala Glu Ala Met Glu Val Ala Ser Gln
        195                 200                 205

Ala Arg Gln Met Val Gln Ala Met Arg Thr Ile Gly Thr His Pro Ser
    210                 215                 220

Ser Ser Ala Gly Leu Lys Asn Asp Leu Leu Glu Asn Leu Gln Ala Tyr
225                 230                 235                 240

Gln Lys Arg Met Gly Val Gln Met Gln Arg Phe Lys Xaa
                245                 250
```

(2) INFORMATION FOR SEQ ID NO:4:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:4:

ATACCCGCGG CAGTCTTCTA ACCGAGGTCG 30

(2) INFORMATION FOR SEQ ID NO:5:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

CCCCACGTCT ACGTTGCCAA GTTCACTCTC GAGATA 36

(2) INFORMATION FOR SEQ ID NO:6:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:6:

CTCGAGAATT CATGGCCGAG GAAGCTT 27

(2) INFORMATION FOR SEQ ID NO:7:

(i) SEQUENCE CHARACTERISTICS:
(A) LENGTH: 1998 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:7:

ATGGCCGAAG AAGCTTTCGA CCTCTGGAAC GAATGCGCCA AAGCCTGCGT GCTCGACCTC 60

AAGGACGGCG TGCGTTCCAG CCGCATGAGC GTCGACCCGG CCATCGCCGA CACCAACGGC 120

CAGGGCGTGC TGCACTACTC CATGGTCCTG GAGGGCGGCA ACGACGCGCT CAAGCTGGCC 180

ATCGACAACG CCCTCAGCAT CACCAGCGAC GGCCTGACCA TCCGCCTCGA AGGCGGCGTC 240

GAGCCGAACA AGCCGGTGCG CTACAGCTAC ACGCGCCAGG CGCGCGGCAG TTGGTCGCTG 300

AACTGGCTGG TACCGATCGG CCACGAGAAG CCCTCGAACA TCAAGGTGTT CATCCACGAA 360

CTGAACGCCG GCAACCAGCT CAGCCACATG TCGCCGATCT ACACCATCGA GATGGGCGAC 420

GAGTTGCTGG CGAAGCTGGC GCGCGATGCC ACCTTCTTCG TCAGGGCGCA CGAGAGCAAC 480

GAGATGCAGC CGACGCTCGC CATCAGCCAT GCCGGGGTCA GCGTGGTCAT GGCCCAGACC 540

CAGCCGCGCC GGGAAAAGCG CTGGAGCGAA TGGGCCAGCG GCAAGGTGTT GTGCCTGCTC 600

GACCCGCTGG ACGGGGTCTA CAACTACCTC GCCCAGCAAC GCTGCAACCT CGACGATACC 660

TGGGAAGGCA AGATCTACCG GGTGCTCGCC GGCAACCCGG CGAAGCATGA CCTGGACATC 720

AAACCCACGG TCATCAGTCA TCGCCTGCAC TTTCCCGAGG GCGGCAGCCT GGCCGCGCTG 780

ACCGCGCACC AGGCTTGCCA CCTGCCGCTG GAGACTTTCA CCCGTCATCG CCAGCCGCGC 840

GGCTGGGAAC AACTGGAGCA GTGCGGCTAT CCGGTGCAGC GGCTGGTCGC CCTCTACCTG 900

GCGGCGCGGC TGTCGTGGAA CCAGGTCGAC CAGGTGATCC GCAACGCCCT GGCCAGCCCC 960

GGCAGCGGCG GCGACCTGGG CGAAGCGATC CGCGAGCAGC CGGAGCAGGC CCGTCTGGCC 1020

CTGACCCTGG CCGCCGCCGA GAGCGAGCGC TTCGTCCGGC AGGGCACCGG CAACGACGAG 1080

GCCGGCGCGG CCAACGCCGA CGTGGTGAGC CTGACCTGCC CGGTCGCCGC CGGTGAATGC 1140

GCGGGCCCGG CGGACAGCGG CGACGCCCTG CTGGAGCGCA ACTATCCCAC TGGCGCGGAG 1200

TTCCTCGGCG ACGGCGGCGA CGTCAGCTTC AGCACCCGCG GCAGTCTTCT AACCGAGGTC 1260

GAAACGTACG TTCTCTCTAT CATCCCGTCA GGCCCCCTCA AAGCCGAGAT CGCACAGAGA 1320

CTTGAAGATG TCTTTGCAGG GAAGAACACC GATCTTGAGG TTCTCATGGA ATGGCTAAAG 1380

ACAAGACCAA TCCTGTCACC TCTGACTAAG GGGATTTTAG GATTTGTGTT CACGCTCACC 1440

GTGCCCAGTG AGCGAGGACT GCAGCGTAGA CGCTTTGTCC AAAATGCCCT TAATGGGAAC 1500

GGGGATCCAA ATAACATGGA CAAAGCAGTT AAACTGTATA GGAAGCTCAA GAGGGAGATA 1560

ACATTCCATG GGGCCAAAGA AATCTCACTC AGTTATTCTG CTGGTGCACT TGCCAGTTGT 1620

ATGGGCCTCA TATACAACAG GATGGGGGCT GTGACCACTG AAGTGGCATT TGGCCTGGTA 1680

TGTGCAACCT GTGAACAGAT TGCTGACTCC CAGCATCGGT CTCATAGGCA AATGGTGACA 1740

ACAACCAACC CACTAATCAG ACATGAGAAC AGAATGGTTT TAGCCAGCAC TACAGCTAAG 1800

GCTATGGAGC AAATGGCTGG ATCGAGTGAG CAAGCAGCAG AGGCCATGGA GGTTGCTAGT 1860

CAGGCTAGGC AAATGGTGCA AGCGATGAGA ACCATTGGGA CTCATCCTAG CTCCAGTGCT 1920

GGTCTGAAAA ATGATCTTCT TGAAAATTTG CAGGCCTATC AGAAACGAAT GGGGGTGCAG 1980

ATGCAACGGT TCAAGTGA 1998

(2) INFORMATION FOR SEQ ID NO:8:

(i) SEQUENCE CHARACTERISTICS:
(A) LENGTH: 666 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:8:

```
Met Ala Glu Glu Ala Phe Asp Leu Trp Asn Glu Cys Ala Lys Ala Cys
1               5                   10                  15

Val Leu Asp Leu Lys Asp Gly Val Arg Ser Ser Arg Met Ser Val Asp
            20                  25                  30

Pro Ala Ile Ala Asp Thr Asn Gly Gln Gly Val Leu His Tyr Ser Met
        35                  40                  45

Val Leu Glu Gly Gly Asn Asp Ala Leu Lys Leu Ala Ile Asp Asn Ala
    50                  55                  60

Leu Ser Ile Thr Ser Asp Gly Leu Thr Ile Arg Leu Glu Gly Gly Val
65                  70                  75                  80

Glu Pro Asn Lys Pro Val Arg Tyr Ser Tyr Thr Arg Gln Ala Arg Gly
                85                  90                  95

Ser Trp Ser Leu Asn Trp Leu Val Pro Ile Gly His Glu Lys Pro Ser
            100                 105                 110

Asn Ile Lys Val Phe Ile His Glu Leu Asn Ala Gly Asn Gln Leu Ser
        115                 120                 125

His Met Ser Pro Ile Tyr Thr Ile Glu Met Gly Asp Glu Leu Leu Ala
    130                 135                 140

Lys Leu Ala Arg Asp Ala Thr Phe Phe Val Arg Ala His Glu Ser Asn
145                 150                 155                 160

Glu Met Gln Pro Thr Leu Ala Ile Ser His Ala Gly Val Ser Val Val
                165                 170                 175

Met Ala Gln Thr Gln Pro Arg Arg Glu Lys Arg Trp Ser Glu Trp Ala
            180                 185                 190
```

Ser Gly Lys Val Leu Cys Leu Leu Asp Pro Leu Asp Gly Val Tyr Asn
195 200 205

Tyr Leu Ala Gln Gln Arg Cys Asn Leu Asp Asp Thr Trp Glu Gly Lys
210 215 220

Ile Tyr Arg Val Leu Ala Gly Asn Pro Ala Lys His Asp Leu Asp Ile
225 230 235 240

Lys Pro Thr Val Ile Ser His Arg Leu His Phe Pro Glu Gly Gly Ser
245 250 255

Leu Ala Ala Leu Thr Ala His Gln Ala Cys His Leu Pro Leu Glu Thr
260 265 270

Phe Thr Arg His Arg Gln Pro Arg Gly Trp Glu Gln Leu Glu Gln Cys
275 280 285

Gly Tyr Pro Val Gln Arg Leu Val Ala Leu Tyr Leu Ala Ala Arg Leu
290 295 300

Ser Trp Asn Gln Val Asp Gln Val Ile Arg Asn Ala Leu Ala Ser Pro
305 310 315 320

Gly Ser Gly Gly Asp Leu Gly Glu Ala Ile Arg Glu Gln Pro Glu Gln
325 330 335

Ala Arg Leu Ala Leu Thr Leu Ala Ala Ala Glu Ser Glu Arg Phe Val
340 345 350

Arg Gln Gly Thr Gly Asn Asp Glu Ala Gly Ala Ala Asn Ala Asp Val
355 360 365

Val Ser Leu Thr Cys Pro Val Ala Ala Gly Glu Cys Ala Gly Pro Ala
370 375 380

Asp Ser Gly Asp Ala Leu Leu Glu Arg Asn Tyr Pro Thr Gly Ala Glu
385 390 395 400

Phe Leu Gly Asp Gly Gly Asp Val Ser Phe Ser Thr Arg Gly Ser Leu
405 410 415

Leu Thr Glu Val Glu Thr Tyr Val Leu Ser Ile Ile Pro Ser Gly Pro
420 425 430

Leu Lys Ala Glu Ile Ala Gln Arg Leu Glu Asp Val Phe Ala Gly Lys
435 440 445

```
Asn Thr Asp Leu Glu Val Leu Met Glu Trp Leu Lys Thr Arg Pro Ile
    450                 455                 460

Leu Ser Pro Leu Thr Lys Gly Ile Leu Gly Phe Val Phe Thr Leu Thr
465                 470                 475                 480

Val Pro Ser Glu Arg Gly Leu Gln Arg Arg Arg Phe Val Gln Asn Ala
                485                 490                 495

Leu Asn Gly Asn Gly Asp Pro Asn Asn Met Asp Lys Ala Val Lys Leu
            500                 505                 510

Tyr Arg Lys Leu Lys Arg Glu Ile Thr Phe His Gly Ala Lys Glu Ile
        515                 520                 525

Ser Leu Ser Tyr Ser Ala Gly Ala Leu Ala Ser Cys Met Gly Leu Ile
    530                 535                 540

Tyr Asn Arg Met Gly Ala Val Thr Thr Glu Val Ala Phe Gly Leu Val
545                 550                 555                 560

Cys Ala Thr Cys Glu Gln Ile Ala Asp Ser Gln His Arg Ser His Arg
                565                 570                 575

Gln Met Val Thr Thr Thr Asn Pro Leu Ile Arg His Glu Asn Arg Met
            580                 585                 590

Val Leu Ala Ser Thr Thr Ala Lys Ala Met Glu Gln Met Ala Gly Ser
        595                 600                 605

Ser Glu Gln Ala Ala Glu Ala Met Glu Val Ala Ser Gln Ala Arg Gln
    610                 615                 620

Met Val Gln Ala Met Arg Thr Ile Gly Thr His Pro Ser Ser Ser Ala
625                 630                 635                 640

Gly Leu Lys Asn Asp Leu Leu Glu Asn Leu Gln Ala Tyr Gln Lys Arg
                645                 650                 655

Met Gly Val Gln Met Gln Arg Phe Lys Xaa
            660                 665
```

(2) INFORMATION FOR SEQ ID NO:9:

(i) SEQUENCE CHARACTERISTICS:
- (A) LENGTH: 52 base pairs
- (B) TYPE: nucleic acid
- (C) STRANDEDNESS: single
- (D) TOPOLOGY: linear

(ii) MOLECULE TYPE: DNA (genomic)

(xi) SEQUENCE DESCRIPTION: SEQ ID NO:9:

CTAGAAATAA TTTTGTTTAA CTTTAAGAAG GAGATATACA TATGGCCGAA GA 52

(2) INFORMATION FOR SEQ ID NO:10:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:10:

ATACCCGCGG CAAGGGGATT TTAGGATTTG TG 32

(2) INFORMATION FOR SEQ ID NO:11:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:11:

ATAGAGCTCT CACACGGTGA GCGTGAACAC AAATCC 36

(2) INFORMATION FOR SEQ ID NO:12:

(i) SEQUENCE CHARACTERISTICS:
(A) LENGTH: 52 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:12:

CCGCGGCAAG GGGATTTTAG GATTTGTGTT CACGCTCACC GTGTGAGAGC TC 52

(2) INFORMATION FOR SEQ ID NO:13:

(i) SEQUENCE CHARACTERISTICS:
(A) LENGTH: 52 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:13:

CTAGAAATAA TTTTGTTTAA CTTTAAGAAG GAGATATACA TATGGCCGAA GA 52

(2) INFORMATION FOR SEQ ID NO:14:

(i) SEQUENCE CHARACTERISTICS:
(A) LENGTH: 1281 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:14:

ATGGCCGAGG AAGCTTTCGA CCTCTGGAAC GAATGCGCCA AAGCCTGCGT GCTCGACCTC 60

AAGGACGGCG TGCGTTCCAG CCGCATGAGC GTCGACCCGG CCATCGCCGA CACCAACGGC 120

CAGGGCGTGC TGCACTACTC CATGGTCCTG GAGGGCGGCA ACGACGCGCT CAAGCTGGCC 180

ATCGACAACG CCCTCAGCAT CACCAGCGAC GGCCTGACCA TCCGCCTCGA AGGCGGCGTC 240

GAGCCGAACA AGCCGGTGCG CTACAGCTAC ACGCGCCAGG CGCGCGGCAG TTGGTCGCTG 300

```
AACTGGCTGG TACCGATCGG CCACGAGAAG CCCTCGAACA TCAAGGTGTT CATCCACGAA    360
CTGAACGCCG GCAACCAGCT CAGCCACATG TCGCCGATCT ACACCATCGA GATGGGCGAC    420
GAGTTGCTGG CGAAGCTGGC GCGCGATGCC ACCTTCTTCG TCAGGGCGCA CGAGAGCAAC    480
GAGATGCAGC CGACGCTCGC CATCAGCCAT GCCGGGGTCA GCGTGGTCAT GGCCCAGACC    540
CAGCCGCGCC GGGAAAAGCG CTGGAGCGAA TGGGCCAGCG GCAAGGTGTT GTGCCTGCTC    600
GACCCGCTGG ACGGGGTCTA CAACTACCTC GCCCAGCAAC GCTGCAACCT CGACGATACC    660
TGGGAAGGCA AGATCTACCG GGTGCTCGCC GGCAACCCGG CGAAGCATGA CCTGGACATC    720
AAACCCACGG TCATCAGTCA TCGCCTGCAC TTTCCCGAGG GCGGCAGCCT GGCCGCGCTG    780
ACCGCGCACC AGGCTTGCCA CCTGCCGCTG GAGACTTTCA CCCGTCATCG CCAGCCGCGC    840
GGCTGGGAAC AACTGGAGCA GTGCGGCTAT CCGGTGCAGC GGCTGGTCGC CCTCTACCTG    900
GCGGCGCGGC TGTCGTGGAA CCAGGTCGAC CAGGTGATCC GCAACGCCCT GGCCAGCCCC    960
GGCAGCGGCG GCGACCTGGG CGAAGCGATC CGCGAGCAGC CGGAGCAGGC CCGTCTGGCC   1020
CTGACCCTGG CCGCCGCCGA GAGCGAGCGC TTCGTCCGGC AGGGCACCGG CAACGACGAG   1080
GCCGGCGCGG CCAACGCCGA CGTGGTGAGC CTGACCTGCC CGGTCGCCGC CGGTGAATGC   1140
GCGGGCCCGG CGGACAGCGG CGACGCCCTG CTGGAGCGCA ACTATCCCAC TGGCGCGGAG   1200
TTCCTCGGCG ACGGCGGCGA CGTCAGCTTC AGCACCCGCG GCAAGGGGAT TTTAGGATTT   1260
GTGTTCACGC TCACCGTGTG A                                              1281
```

(2) INFORMATION FOR SEQ ID NO:15:

(i) SEQUENCE CHARACTERISTICS:
- (A) LENGTH: 427 amino acids
- (B) TYPE: amino acid
- (C) STRANDEDNESS: single
- (D) TOPOLOGY: linear

(ii) MOLECULE TYPE: protein

(xi) SEQUENCE DESCRIPTION: SEQ ID NO:15:

Met Ala Glu Glu Ala Phe Asp Leu Trp Asn Glu Cys Ala Lys Ala Cys
1 5 10 15

Val Leu Asp Leu Lys Asp Gly Val Arg Ser Ser Arg Met Ser Val Asp
20 25 30

Pro Ala Ile Ala Asp Thr Asn Gly Gln Gly Val Leu His Tyr Ser Met
35 40 45

Val Leu Glu Gly Gly Asn Asp Ala Leu Lys Leu Ala Ile Asp Asn Ala
50 55 60

Leu Ser Ile Thr Ser Asp Gly Leu Thr Ile Arg Leu Glu Gly Gly Val
65 70 75 80

Glu Pro Asn Lys Pro Val Arg Tyr Ser Tyr Thr Arg Gln Ala Arg Gly
85 90 95

Ser Trp Ser Leu Asn Trp Leu Val Pro Ile Gly His Glu Lys Pro Ser
100 105 110

Asn Ile Lys Val Phe Ile His Glu Leu Asn Ala Gly Asn Gln Leu Ser
115 120 125

His Met Ser Pro Ile Tyr Thr Ile Glu Met Gly Asp Glu Leu Leu Ala
130 135 140

Lys Leu Ala Arg Asp Ala Thr Phe Phe Val Arg Ala His Glu Ser Asn
145 150 155 160

Glu Met Gln Pro Thr Leu Ala Ile Ser His Ala Gly Val Ser Val Val
165 170 175

Met Ala Gln Thr Gln Pro Arg Arg Glu Lys Arg Trp Ser Glu Trp Ala
180 185 190

Ser Gly Lys Val Leu Cys Leu Leu Asp Pro Leu Asp Gly Val Tyr Asn
195 200 205

Tyr Leu Ala Gln Gln Arg Cys Asn Leu Asp Asp Thr Trp Glu Gly Lys
210 215 220

Ile Tyr Arg Val Leu Ala Gly Asn Pro Ala Lys His Asp Leu Asp Ile
225 230 235 240

Lys Pro Thr Val Ile Ser His Arg Leu His Phe Pro Glu Gly Gly Ser
245 250 255

```
Leu Ala Ala Leu Thr Ala His Gln Ala Cys His Leu Pro Leu Glu Thr
            260                 265                 270

Phe Thr Arg His Arg Gln Pro Arg Gly Trp Glu Gln Leu Glu Gln Cys
        275                 280                 285

Gly Tyr Pro Val Gln Arg Leu Val Ala Leu Tyr Leu Ala Ala Arg Leu
    290                 295                 300

Ser Trp Asn Gln Val Asp Gln Val Ile Arg Asn Ala Leu Ala Ser Pro
305                 310                 315                 320

Gly Ser Gly Gly Asp Leu Gly Glu Ala Ile Arg Glu Gln Pro Glu Gln
                325                 330                 335

Ala Arg Leu Ala Leu Thr Leu Ala Ala Ala Glu Ser Glu Arg Phe Val
            340                 345                 350

Arg Gln Gly Thr Gly Asn Asp Glu Ala Gly Ala Ala Asn Ala Asp Val
        355                 360                 365

Val Ser Leu Thr Cys Pro Val Ala Ala Gly Glu Cys Ala Gly Pro Ala
    370                 375                 380

Asp Ser Gly Asp Ala Leu Leu Glu Arg Asn Tyr Pro Thr Gly Ala Glu
385                 390                 395                 400

Phe Leu Gly Asp Gly Gly Asp Val Ser Phe Ser Thr Arg Gly Lys Gly
                405                 410                 415

Ile Leu Gly Phe Val Phe Thr Leu Thr Val Xaa
            420                 425
```

(2) INFORMATION FOR SEQ ID NO:16:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:16:

GGCTGATAAT AGAGCTCG 18

(2) INFORMATION FOR SEQ ID NO:17:

(i) SEQUENCE CHARACTERISTICS:
(A) LENGTH: 1245 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:17:

ATGGCCGAGG AAGCTTTCGA CCTCTGGAAC GAATGCGCCA AAGCCTGCGT GCTCGACCTC 60

AAGGACGGCG TGCGTTCCAG CCGCATGAGC GTCGACCCGG CCATCGCCGA CACCAACGGC 120

CAGGGCGTGC TGCACTACTC CATGGTCCTG GAGGGCGGCA ACGACGCGCT CAAGCTGGCC 180

ATCGACAACG CCCTCAGCAT CACCAGCGAC GGCCTGACCA TCCGCCTCGA AGGCGGCGTC 240

GAGCCGAACA AGCCGGTGCG CTACAGCTAC ACGCGCCAGG CGCGCGGCAG TTGGTCGCTG 300

AACTGGCTGG TACCGATCGG CCACGAGAAG CCCTCGAACA TCAAGGTGTT CATCCACGAA 360

CTGAACGCCG GCAACCAGCT CAGCCACATG TCGCCGATCT ACACCATCGA GATGGGCGAC 420

GAGTTGCTGG CGAAGCTGGC GCGCGATGCC ACCTTCTTCG TCAGGGCGCA CGAGAGCAAC 480

GAGATGCAGC CGACGCTCGC CATCAGCCAT GCCGGGGTCA GCGTGGTCAT GGCCCAGACC 540

CAGCCGCGCC GGGAAAAGCG CTGGAGCGAA TGGGCCAGCG GCAAGGTGTT GTGCCTGCTC 600

GACCCGCTGG ACGGGGTCTA CAACTACCTC GCCCAGCAAC GCTGCAACCT CGACGATACC 660

TGGGAAGGCA AGATCTACCG GGTGCTCGCC GGCAACCCGG CGAAGCATGA CCTGGACATC 720

AAACCCACGG TCATCAGTCA TCGCCTGCAC TTTCCCGAGG GCGGCAGCCT GGCCGCGCTG 780

ACCGCGCACC AGGCTTGCCA CCTGCCGCTG GAGACTTTCA CCCGTCATCG CCAGCCGCGC 840

GGCTGGGAAC AACTGGAGCA GTGCGGCTAT CCGGTGCAGC GGCTGGTCGC CCTCTACCTG 900

GCGGCGCGGC TGTCGTGGAA CCAGGTCGAC CAGGTGATCC GCAACGCCCT GGCCAGCCCC 960

GGCAGCGGCG GCGACCTGGG CGAAGCGATC CGCGAGCAGC CGGAGCAGGC CCGTCTGGCC 1020

CTGACCCTGG CCGCCGCCGA GAGCGAGCGC TTCGTCCGGC AGGGCACCGG CAACGACGAG 1080

GCCGGCGCGG CCAACGCCGA CGTGGTGAGC CTGACCTGCC CGGTCGCCGC CGGTGAATGC 1140

GCGGGCCCGG CGGACAGCGG CGACGCCCTG CTGGAGCGCA ACTATCCCAC TGGCGCGGAG 1200

TTCCTCGGCG ACGGCGGCGA CGTCAGCTTC AGCACCCGCG GCTGA 1245

(2) INFORMATION FOR SEQ ID NO:18:

(i) SEQUENCE CHARACTERISTICS:
(A) LENGTH: 415 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:18:

```
Met Ala Glu Glu Ala Phe Asp Leu Trp Asn Glu Cys Ala Lys Ala Cys
1               5                   10                  15

Val Leu Asp Leu Lys Asp Gly Val Arg Ser Ser Arg Met Ser Val Asp
            20                  25                  30

Pro Ala Ile Ala Asp Thr Asn Gly Gln Gly Val Leu His Tyr Ser Met
        35                  40                  45

Val Leu Glu Gly Gly Asn Asp Ala Leu Lys Leu Ala Ile Asp Asn Ala
    50                  55                  60

Leu Ser Ile Thr Ser Asp Gly Leu Thr Ile Arg Leu Glu Gly Gly Val
65                  70                  75                  80

Glu Pro Asn Lys Pro Val Arg Tyr Ser Tyr Thr Arg Gln Ala Arg Gly
                85                  90                  95

Ser Trp Ser Leu Asn Trp Leu Val Pro Ile Gly His Glu Lys Pro Ser
            100                 105                 110

Asn Ile Lys Val Phe Ile His Glu Leu Asn Ala Gly Asn Gln Leu Ser
        115                 120                 125

His Met Ser Pro Ile Tyr Thr Ile Glu Met Gly Asp Glu Leu Leu Ala
    130                 135                 140
```

Lys Leu Ala Arg Asp Ala Thr Phe Phe Val Arg Ala His Glu Ser Asn
145 150 155 160

Glu Met Gln Pro Thr Leu Ala Ile Ser His Ala Gly Val Ser Val Val
165 170 175

Met Ala Gln Thr Gln Pro Arg Arg Glu Lys Arg Trp Ser Glu Trp Ala
180 185 190

Ser Gly Lys Val Leu Cys Leu Leu Asp Pro Leu Asp Gly Val Tyr Asn
195 200 205

Tyr Leu Ala Gln Gln Arg Cys Asn Leu Asp Asp Thr Trp Glu Gly Lys
210 215 220

Ile Tyr Arg Val Leu Ala Gly Asn Pro Ala Lys His Asp Leu Asp Ile
225 230 235 240

Lys Pro Thr Val Ile Ser His Arg Leu His Phe Pro Glu Gly Gly Ser
245 250 255

Leu Ala Ala Leu Thr Ala His Gln Ala Cys His Leu Pro Leu Glu Thr
260 265 270

Phe Thr Arg His Arg Gln Pro Arg Gly Trp Glu Gln Leu Glu Gln Cys
275 280 285

Gly Tyr Pro Val Gln Arg Leu Val Ala Leu Tyr Leu Ala Ala Arg Leu
290 295 300

Ser Trp Asn Gln Val Asp Gln Val Ile Arg Asn Ala Leu Ala Ser Pro
305 310 315 320

Gly Ser Gly Gly Asp Leu Gly Glu Ala Ile Arg Glu Gln Pro Glu Gln
325 330 335

Ala Arg Leu Ala Leu Thr Leu Ala Ala Ala Glu Ser Glu Arg Phe Val
340 345 350

Arg Gln Gly Thr Gly Asn Asp Glu Ala Gly Ala Ala Asn Ala Asp Val
355 360 365

Val Ser Leu Thr Cys Pro Val Ala Ala Gly Glu Cys Ala Gly Pro Ala
370 375 380

Asp Ser Gly Asp Ala Leu Leu Glu Arg Asn Tyr Pro Thr Gly Ala Glu
385 390 395 400

Phe Leu Gly Asp Gly Gly Asp Val Ser Phe Ser Thr Arg Gly Xaa
405 410 415

(2) INFORMATION FOR SEQ ID NO:19:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:19:

TCGAGCCGCC ACCATGGCCG AGGAA 25

(2) INFORMATION FOR SEQ ID NO:20:

(i) SEQUENCE CHARACTERISTICS:
(A) LENGTH: 46 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:20:

GACCCGCTAG CACCCGGGAA ACCGCCGCGC GAGGACCTGA AGTAAG 46

(2) INFORMATION FOR SEQ ID NO:21:

(i) SEQUENCE CHARACTERISTICS:
(A) LENGTH: 1956 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:21:

```
ATGCACCTGA TACCCCATTG GATCCCCCTG GTCGCCAGCC TCGGCCTGCT CGCCGGCGGC     60
TCGTCCGCGT CCGCCGCCGA GGAAGCTTTC GACCTCTGGA ACGAATGCGC CAAAGCCTGC    120
GTGCTCGACC TCAAGGACGG CGTGCGTTCC AGCCGCATGA GCGTCGACCC GGCCATCGCC    180
GACACCAACG GCCAGGGCGT GCTGCACTAC TCCATGGTCC TGGAGGGCGG CAACGACGCG    240
CTCAAGCTGG CCATCGACAA CGCCCTCAGC ATCACCAGCG ACGGCCTGAC CATCCGCCTC    300
GAAGGCGGCG TCGAGCCGAA CAAGCCGGTG CGCTACAGCT ACACGCGCCA GGCGCGCGGC    360
AGTTGGTCGC TGAACTGGCT GGTACCGATC GGCCACGAGA AGCCCTCGAA CATCAAGGTG    420
TTCATCCACG AACTGAACGC CGGCAACCAG CTCAGCCACA TGTCGCCGAT CTACACCATC    480
GAGATGGGCG ACGAGTTGCT GGCGAAGCTG GCGCGCGATG CCACCTTCTT CGTCAGGGCG    540
CACGAGAGCA ACGAGATGCA GCCGACGCTC GCCATCAGCC ATGCCGGGGT CAGCGTGGTC    600
ATGGCCCAGA CCCAGCCGCG CCGGGAAAAG CGCTGGAGCG AATGGGCCAG CGGCAAGGTG    660
TTGTGCCTGC TCGACCCGCT GGACGGGGTC TACAACTACC TCGCCCAGCA ACGCTGCAAC    720
CTCGACGATA CCTGGGAAGG CAAGATCTAC CGGGTGCTCG CCGGCAACCC GGCGAAGCAT    780
GACCTGGACA TCAAACCCAC GGTCATCAGT CATCGCCTGC ACTTTCCCGA GGGCGGCAGC    840
CTGGCCGCGC TGACCGCGCA CCAGGCTTGC CACCTGCCGC TGGAGACTTT CACCCGTCAT    900
CGCCAGCCGC GCGGCTGGGA ACAACTGGAG CAGTGCGGCT ATCCGGTGCA GCGGCTGGTC    960
GCCCTCTACC TGGCGGCGCG GCTGTCGTGG AACCAGGTCG ACCAGGTGAT CCGCAACGCC   1020
CTGGCCAGCC CCGGCAGCGG CGGCGACCTG GGCGAAGCGA TCCGCGAGCA GCCGGAGCAG   1080
GCCCGTCTGG CCCTGACCCT GGCCGCCGCC GAGAGCGAGC GCTTCGTCCG GCAGGGCACC   1140
GGCAACGACG AGGCCGGCGC GGCCAACGCC GACGTGGTGA GCCTGACCTG CCCGGTCGCC   1200
GCCGGTGAAT GCGCGGGCCC GGCGGACAGC GGCGACGCCC TGCTGGAGCG CAACTATCCC   1260
ACTGGCGCGG AGTTCCTCGG CGACGGCGGC GACGTCAGCT TCAGCACCCG CGGCACGCAG   1320
AACTGGACGG TGGAGCGGCT GCTCCAGGCG CACCGCCAAC TGGAGGAGCG CGGCTATGTG   1380
```

TTCGTCGGCT ACCACGGCAC CTTCCTCGAA GCGGCGCAAA GCATCGTCTT CGGCGGGGTG 1440

CGCGCGCGCA GCCAGGACCT CGACGCGATC TGGCGCGGTT TCTATATCGC CGGCGATCCG 1500

GCGCTGGCCT ACGGCTACGC CCAGGACCAG GAACCCGACG CACGCGGCCG GATCCGCAAC 1560

GGTGCCCTGC TGCGGGTCTA TGTGCCGCGC TCGAGCCTGC CGGGCTTCTA CCGCACCAGC 1620

CTGACCCTGG CCGCGCCGGA GGCGGCGGGC GAGGTCGAAC GGCTGATCGG CCATCCGCTG 1680

CCGCTGCGCC TGGACGCCAT CACCGGCCCC GAGGAGGAAG GCGGGCGCCT GGAGACCATT 1740

CTCGGCTGGC CGCTGGCCGA GCGCACCGTG GTGATTCCCT CGGCGATCCC CACCGACCCG 1800

CGCAACGTCG GCGGCGACCT CGACCCGTCC AGCATCCCCG ACAAGGAACA GGCGATCAGC 1860

GCCCTGCCGG ACTACGCCAG CCAGCCCGGC AAACCGCCGC GCGAGGACCC GCTAGCACCC 1920

GGGAAACCGC CGCGCGAGGA CCTGAAGTAA GAATTC 1956

(2) INFORMATION FOR SEQ ID NO:22:

(i) SEQUENCE CHARACTERISTICS:
(A) LENGTH: 652 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:22:

Met His Leu Ile Pro His Trp Ile Pro Leu Val Ala Ser Leu Gly Leu
1 5 10 15

Leu Ala Gly Gly Ser Ser Ala Ser Ala Ala Glu Glu Ala Phe Asp Leu
20 25 30

Trp Asn Glu Cys Ala Lys Ala Cys Val Leu Asp Leu Lys Asp Gly Val
35 40 45

Arg Ser Ser Arg Met Ser Val Asp Pro Ala Ile Ala Asp Thr Asn Gly
50 55 60

Gln Gly Val Leu His Tyr Ser Met Val Leu Glu Gly Gly Asn Asp Ala
65 70 75 80

Leu Lys Leu Ala Ile Asp Asn Ala Leu Ser Ile Thr Ser Asp Gly Leu
85 90 95

Thr Ile Arg Leu Glu Gly Gly Val Glu Pro Asn Lys Pro Val Arg Tyr
100 105 110

Ser Tyr Thr Arg Gln Ala Arg Gly Ser Trp Ser Leu Asn Trp Leu Val
115 120 125

Pro Ile Gly His Glu Lys Pro Ser Asn Ile Lys Val Phe Ile His Glu
130 135 140

Leu Asn Ala Gly Asn Gln Leu Ser His Met Ser Pro Ile Tyr Thr Ile
145 150 155 160

Glu Met Gly Asp Glu Leu Leu Ala Lys Leu Ala Arg Asp Ala Thr Phe
165 170 175

Phe Val Arg Ala His Glu Ser Asn Glu Met Gln Pro Thr Leu Ala Ile
180 185 190

Ser His Ala Gly Val Ser Val Val Met Ala Gln Thr Gln Pro Arg Arg
195 200 205

Glu Lys Arg Trp Ser Glu Trp Ala Ser Gly Lys Val Leu Cys Leu Leu
210 215 220

Asp Pro Leu Asp Gly Val Tyr Asn Tyr Leu Ala Gln Gln Arg Cys Asn
225 230 235 240

Leu Asp Asp Thr Trp Glu Gly Lys Ile Tyr Arg Val Leu Ala Gly Asn
245 250 255

Pro Ala Lys His Asp Leu Asp Ile Lys Pro Thr Val Ile Ser His Arg
260 265 270

Leu His Phe Pro Glu Gly Gly Ser Leu Ala Ala Leu Thr Ala His Gln
275 280 285

Ala Cys His Leu Pro Leu Glu Thr Phe Thr Arg His Arg Gln Pro Arg
290 295 300

Gly Trp Glu Gln Leu Glu Gln Cys Gly Tyr Pro Val Gln Arg Leu Val
305 310 315 320

Ala Leu Tyr Leu Ala Ala Arg Leu Ser Trp Asn Gln Val Asp Gln Val
325 330 335

```
Ile Arg Asn Ala Leu Ala Ser Pro Gly Ser Gly Gly Asp Leu Gly Glu
            340                 345                 350

Ala Ile Arg Glu Gln Pro Glu Gln Ala Arg Leu Ala Leu Thr Leu Ala
        355                 360                 365

Ala Ala Glu Ser Glu Arg Phe Val Arg Gln Gly Thr Gly Asn Asp Glu
    370                 375                 380

Ala Gly Ala Ala Asn Ala Asp Val Val Ser Leu Thr Cys Pro Val Ala
385                 390                 395                 400

Ala Gly Glu Cys Ala Gly Pro Ala Asp Ser Gly Asp Ala Leu Leu Glu
                405                 410                 415

Arg Asn Tyr Pro Thr Gly Ala Glu Phe Leu Gly Asp Gly Gly Asp Val
            420                 425                 430

Ser Phe Ser Thr Arg Gly Thr Gln Asn Trp Thr Val Glu Arg Leu Leu
        435                 440                 445

Gln Ala His Arg Gln Leu Glu Glu Arg Gly Tyr Val Phe Val Gly Tyr
    450                 455                 460

His Gly Thr Phe Leu Glu Ala Ala Gln Ser Ile Val Phe Gly Gly Val
465                 470                 475                 480

Arg Ala Arg Ser Gln Asp Leu Asp Ala Ile Trp Arg Gly Phe Tyr Ile
                485                 490                 495

Ala Gly Asp Pro Ala Leu Ala Tyr Gly Tyr Ala Gln Asp Gln Glu Pro
            500                 505                 510

Asp Ala Arg Gly Arg Ile Arg Asn Gly Ala Leu Leu Arg Val Tyr Val
        515                 520                 525

Pro Arg Ser Ser Leu Pro Gly Phe Tyr Arg Thr Ser Leu Thr Leu Ala
    530                 535                 540

Ala Pro Glu Ala Ala Gly Glu Val Glu Arg Leu Ile Gly His Pro Leu
545                 550                 555                 560

Pro Leu Arg Leu Asp Ala Ile Thr Gly Pro Glu Glu Glu Gly Gly Arg
                565                 570                 575

Leu Glu Thr Ile Leu Gly Trp Pro Leu Ala Glu Arg Thr Val Val Ile
            580                 585                 590
```

Pro Ser Ala Ile Pro Thr Asp Pro Arg Asn Val Gly Gly Asp Leu Asp
595 600 605

Pro Ser Ser Ile Pro Asp Lys Glu Gln Ala Ile Ser Ala Leu Pro Asp
610 615 620

Tyr Ala Ser Gln Pro Gly Lys Pro Pro Arg Glu Asp Pro Leu Ala Pro
625 630 635 640

Gly Lys Pro Pro Arg Glu Asp Leu Lys Xaa Glu Phe
645 650

(2) INFORMATION FOR SEQ ID NO:23:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:23:

CCGGGCTGAC TAAGGGGATT TTAGGATTTG TGTTCACGCT CACCGTGC 48

(2) INFORMATION FOR SEQ ID NO:24:

(i) SEQUENCE CHARACTERISTICS:
(A) LENGTH: 2004 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:24:

ATGCACCTGA TACCCCATTG GATCCCCCTG GTCGCCAGCC TCGGCCTGCT CGCCGGCGGC 60

TCGTCCGCGT CCGCCGCCGA GGAAGCTTTC GACCTCTGGA ACGAATGCGC CAAAGCCTGC 120

GTGCTCGACC TCAAGGACGG CGTGCGTTCC AGCCGCATGA GCGTCGACCC GGCCATCGCC 180

```
GACACCAACG GCCAGGGCGT GCTGCACTAC TCCATGGTCC TGGAGGGCGG CAACGACGCG   240
CTCAAGCTGG CCATCGACAA CGCCCTCAGC ATCACCAGCG ACGGCCTGAC CATCCGCCTC   300
GAAGGCGGCG TCGAGCCGAA CAAGCCGGTG CGCTACAGCT ACACGCGCCA GGCGCGCGGC   360
AGTTGGTCGC TGAACTGGCT GGTACCGATC GGCCACGAGA AGCCCTCGAA CATCAAGGTG   420
TTCATCCACG AACTGAACGC CGGCAACCAG CTCAGCCACA TGTCGCCGAT CTACACCATC   480
GAGATGGGCG ACGAGTTGCT GGCGAAGCTG GCGCGCGATG CCACCTTCTT CGTCAGGGCG   540
CACGAGAGCA ACGAGATGCA GCCGACGCTC GCCATCAGCC ATGCCGGGGT CAGCGTGGTC   600
ATGGCCCAGA CCCAGCCGCG CCGGGAAAAG CGCTGGAGCG AATGGGCCAG CGGCAAGGTG   660
TTGTGCCTGC TCGACCCGCT GGACGGGGTC TACAACTACC TCGCCCAGCA ACGCTGCAAC   720
CTCGACGATA CCTGGGAAGG CAAGATCTAC CGGGTGCTCG CCGGCAACCC GGCGAAGCAT   780
GACCTGGACA TCAAACCCAC GGTCATCAGT CATCGCCTGC ACTTTCCCGA GGGCGGCAGC   840
CTGGCCGCGC TGACCGCGCA CCAGGCTTGC CACCTGCCGC TGGAGACTTT CACCCGTCAT   900
CGCCAGCCGC GCGGCTGGGA ACAACTGGAG CAGTGCGGCT ATCCGGTGCA GCGGCTGGTC   960
GCCCTCTACC TGGCGGCGCG GCTGTCGTGG AACCAGGTCG ACCAGGTGAT CCGCAACGCC   1020
CTGGCCAGCC CCGGCAGCGG CGGCGACCTG GGCGAAGCGA TCCGCGAGCA GCCGGAGCAG   1080
GCCCGTCTGG CCCTGACCCT GGCCGCCGCC GAGAGCGAGC GCTTCGTCCG GCAGGGCACC   1140
GGCAACGACG AGGCCGGCGC GGCCAACGCC GACGTGGTGA GCCTGACCTG CCCGGTCGCC   1200
GCCGGTGAAT GCGCGGGCCC GGCGGACAGC GGCGACGCCC TGCTGGAGCG CAACTATCCC   1260
ACTGGCGCGG AGTTCCTCGG CGACGGCGGC GACGTCAGCT TCAGCACCCG CGGCACGCAG   1320
AACTGGACGG TGGAGCGGCT GCTCCAGGCG CACCGCCAAC TGGAGGAGCG CGGCTATGTG   1380
TTCGTCGGCT ACCACGGCAC CTTCCTCGAA GCGGCGCAAA GCATCGTCTT CGGCGGGGTG   1440
CGCGCGCGCA GCCAGGACCT CGACGCGATC TGGCGCGGTT TCTATATCGC CGGCGATCCG   1500
GCGCTGGCCT ACGGCTACGC CCAGGACCAG GAACCCGACG CACGCGGCCG GATCCGCAAC   1560
GGTGCCCTGC TGCGGGTCTA TGTGCCGCGC TCGAGCCTGC CGGGCTTCTA CCGCACCAGC   1620
```

CTGACCCTGG CCGCGCCGGA GGCGGCGGGC GAGGTCGAAC GGCTGATCGG CCATCCGCTG 1680

CCGCTGCGCC TGGACGCCAT CACCGGCCCC GAGGAGGAAG GCGGGCGCCT GGAGACCATT 1740

CTCGGCTGGC CGCTGGCCGA GCGCACCGTG GTGATTCCCT CGGCGATCCC CACCGACCCG 1800

CGCAACGTCG GCGGCGACCT CGACCCGTCC AGCATCCCCG ACAAGGAACA GGCGATCAGC 1860

GCCCTGCCGG ACTACGCCAG CCAGCCCGGC AAACCGCCGC GCGAGGACCC GCTAGCACCC 1920

GGGCTGACTA AGGGGATTTT AGGATTTGTG TTCACGCTCA CCGTGCCCGG GAAACCGCCG 1980

CGCGAGGACC TGAAGTAAGA ATTC 2004

(2) INFORMATION FOR SEQ ID NO:25:

(i) SEQUENCE CHARACTERISTICS:
(A) LENGTH: 668 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:25:

Met His Leu Ile Pro His Trp Ile Pro Leu Val Ala Ser Leu Gly Leu
1 5 10 15

Leu Ala Gly Gly Ser Ser Ala Ser Ala Ala Glu Glu Ala Phe Asp Leu
20 25 30

Trp Asn Glu Cys Ala Lys Ala Cys Val Leu Asp Leu Lys Asp Gly Val
35 40 45

Arg Ser Ser Arg Met Ser Val Asp Pro Ala Ile Ala Asp Thr Asn Gly
50 55 60

Gln Gly Val Leu His Tyr Ser Met Val Leu Glu Gly Gly Asn Asp Ala
65 70 75 80

Leu Lys Leu Ala Ile Asp Asn Ala Leu Ser Ile Thr Ser Asp Gly Leu
85 90 95

Thr Ile Arg Leu Glu Gly Gly Val Glu Pro Asn Lys Pro Val Arg Tyr
100 105 110

Ser Tyr Thr Arg Gln Ala Arg Gly Ser Trp Ser Leu Asn Trp Leu Val
115 120 125

Pro Ile Gly His Glu Lys Pro Ser Asn Ile Lys Val Phe Ile His Glu
130 135 140

Leu Asn Ala Gly Asn Gln Leu Ser His Met Ser Pro Ile Tyr Thr Ile
145 150 155 160

Glu Met Gly Asp Glu Leu Leu Ala Lys Leu Ala Arg Asp Ala Thr Phe
165 170 175

Phe Val Arg Ala His Glu Ser Asn Glu Met Gln Pro Thr Leu Ala Ile
180 185 190

Ser His Ala Gly Val Ser Val Val Met Ala Gln Thr Gln Pro Arg Arg
195 200 205

Glu Lys Arg Trp Ser Glu Trp Ala Ser Gly Lys Val Leu Cys Leu Leu
210 215 220

Asp Pro Leu Asp Gly Val Tyr Asn Tyr Leu Ala Gln Gln Arg Cys Asn
225 230 235 240

Leu Asp Asp Thr Trp Glu Gly Lys Ile Tyr Arg Val Leu Ala Gly Asn
245 250 255

Pro Ala Lys His Asp Leu Asp Ile Lys Pro Thr Val Ile Ser His Arg
260 265 270

Leu His Phe Pro Glu Gly Gly Ser Leu Ala Ala Leu Thr Ala His Gln
275 280 285

Ala Cys His Leu Pro Leu Glu Thr Phe Thr Arg His Arg Gln Pro Arg
290 295 300

Gly Trp Glu Gln Leu Glu Gln Cys Gly Tyr Pro Val Gln Arg Leu Val
305 310 315 320

Ala Leu Tyr Leu Ala Ala Arg Leu Ser Trp Asn Gln Val Asp Gln Val
325 330 335

Ile Arg Asn Ala Leu Ala Ser Pro Gly Ser Gly Gly Asp Leu Gly Glu
340 345 350

Ala Ile Arg Glu Gln Pro Glu Gln Ala Arg Leu Ala Leu Thr Leu Ala
355 360 365

Ala Ala Glu Ser Glu Arg Phe Val Arg Gln Gly Thr Gly Asn Asp Glu
370 375 380

Ala Gly Ala Ala Asn Ala Asp Val Val Ser Leu Thr Cys Pro Val Ala
385 390 395 400

Ala Gly Glu Cys Ala Gly Pro Ala Asp Ser Gly Asp Ala Leu Leu Glu
405 410 415

Arg Asn Tyr Pro Thr Gly Ala Glu Phe Leu Gly Asp Gly Gly Asp Val
420 425 430

Ser Phe Ser Thr Arg Gly Thr Gln Asn Trp Thr Val Glu Arg Leu Leu
435 440 445

Gln Ala His Arg Gln Leu Glu Glu Arg Gly Tyr Val Phe Val Gly Tyr
450 455 460

His Gly Thr Phe Leu Glu Ala Ala Gln Ser Ile Val Phe Gly Gly Val
465 470 475 480

Arg Ala Arg Ser Gln Asp Leu Asp Ala Ile Trp Arg Gly Phe Tyr Ile
485 490 495

Ala Gly Asp Pro Ala Leu Ala Tyr Gly Tyr Ala Gln Asp Gln Glu Pro
500 505 510

Asp Ala Arg Gly Arg Ile Arg Asn Gly Ala Leu Leu Arg Val Tyr Val
515 520 525

Pro Arg Ser Ser Leu Pro Gly Phe Tyr Arg Thr Ser Leu Thr Leu Ala
530 535 540

Ala Pro Glu Ala Ala Gly Glu Val Glu Arg Leu Ile Gly His Pro Leu
545 550 555 560

Pro Leu Arg Leu Asp Ala Ile Thr Gly Pro Glu Glu Glu Gly Gly Arg
565 570 575

Leu Glu Thr Ile Leu Gly Trp Pro Leu Ala Glu Arg Thr Val Val Ile
580 585 590

Pro Ser Ala Ile Pro Thr Asp Pro Arg Asn Val Gly Gly Asp Leu Asp
595 600 605

Pro Ser Ser Ile Pro Asp Lys Glu Gln Ala Ile Ser Ala Leu Pro Asp
610 615 620

```
Tyr Ala Ser Gln Pro Gly Lys Pro Pro Arg Glu Asp Pro Leu Ala Pro
625                 630                 635                 640

Gly Leu Thr Lys Gly Ile Leu Gly Phe Val Phe Thr Leu Thr Val Pro
                645                 650                 655

Gly Lys Pro Pro Arg Glu Asp Leu Lys Xaa Glu Phe
            660                 665
```

(2) INFORMATION FOR SEQ ID NO:26:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:26:

```
GCACCCGGGA TCCCGTCAGG CCCCCTC                                          27
```

(2) INFORMATION FOR SEQ ID NO:27:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:27:

```
GCACCCGGGC TCCCTCTTGA GCTTCCT                                          27
```

(2) INFORMATION FOR SEQ ID NO:28:

(i) SEQUENCE CHARACTERISTICS:
(A) LENGTH: 2238 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:28:

```
ATGCACCTGA TACCCCATTG GATCCCCCTG GTCGCCAGCC TCGGCCTGCT CGCCGGCGGC     60
TCGTCCGCGT CCGCCGCCGA GGAAGCTTTC GACCTCTGGA ACGAATGCGC CAAAGCCTGC    120
GTGCTCGACC TCAAGGACGG CGTGCGTTCC AGCCGCATGA GCGTCGACCC GGCCATCGCC    180
GACACCAACG GCCAGGGCGT GCTGCACTAC TCCATGGTCC TGGAGGGCGG CAACGACGCG    240
CTCAAGCTGG CCATCGACAA CGCCCTCAGC ATCACCAGCG ACGGCCTGAC CATCCGCCTC    300
GAAGGCGGCG TCGAGCCGAA CAAGCCGGTG CGCTACAGCT ACACGCGCCA GGCGCGCGGC    360
AGTTGGTCGC TGAACTGGCT GGTACCGATC GGCCACGAGA AGCCCTCGAA CATCAAGGTG    420
TTCATCCACG AACTGAACGC CGGCAACCAG CTCAGCCACA TGTCGCCGAT CTACACCATC    480
GAGATGGGCG ACGAGTTGCT GGCGAAGCTG GCGCGCGATG CCACCTTCTT CGTCAGGGCG    540
CACGAGAGCA ACGAGATGCA GCCGACGCTC GCCATCAGCC ATGCCGGGGT CAGCGTGGTC    600
ATGGCCCAGA CCCAGCCGCG CCGGGAAAAG CGCTGGAGCG AATGGGCCAG CGGCAAGGTG    660
TTGTGCCTGC TCGACCCGCT GGACGGGGTC TACAACTACC TCGCCCAGCA ACGCTGCAAC    720
CTCGACGATA CCTGGGAAGG CAAGATCTAC CGGGTGCTCG CCGGCAACCC GGCGAAGCAT    780
GACCTGGACA TCAAACCCAC GGTCATCAGT CATCGCCTGC ACTTTCCCGA GGGCGGCAGC    840
CTGGCCGCGC TGACCGCGCA CCAGGCTTGC CACCTGCCGC TGGAGACTTT CACCCGTCAT    900
CGCCAGCCGC GCGGCTGGGA ACAACTGGAG CAGTGCGGCT ATCCGGTGCA GCGGCTGGTC    960
GCCCTCTACC TGGCGGCGCG GCTGTCGTGG AACCAGGTCG ACCAGGTGAT CCGCAACGCC   1020
CTGGCCAGCC CCGGCAGCGG CGGCGACCTG GGCGAAGCGA TCCGCGAGCA GCCGGAGCAG   1080
GCCCGTCTGG CCCTGACCCT GGCCGCCGCC GAGAGCGAGC GCTTCGTCCG GCAGGGCACC   1140
GGCAACGACG AGGCCGGCGC GGCCAACGCC GACGTGGTGA GCCTGACCTG CCCGGTCGCC   1200
GCCGGTGAAT GCGCGGGCCC GGCGGACAGC GGCGACGCCC TGCTGGAGCG CAACTATCCC   1260
```

ACTGGCGCGG AGTTCCTCGG CGACGGCGGC GACGTCAGCT TCAGCACCCG CGGCACGCAG 1320

AACTGGACGG TGGAGCGGCT GCTCCAGGCG CACCGCCAAC TGGAGGAGCG CGGCTATGTG 1380

TTCGTCGGCT ACCACGGCAC CTTCCTCGAA GCGGCGCAAA GCATCGTCTT CGGCGGGGTG 1440

CGCGCGCGCA GCCAGGACCT CGACGCGATC TGGCGCGGTT TCTATATCGC CGGCGATCCG 1500

GCGCTGGCCT ACGGCTACGC CCAGGACCAG GAACCCGACG CACGCGGCCG GATCCGCAAC 1560

GGTGCCCTGC TGCGGGTCTA TGTGCCGCGC TCGAGCCTGC CGGGCTTCTA CCGCACCAGC 1620

CTGACCCTGG CCGCGCCGGA GGCGGCGGGC GAGGTCGAAC GGCTGATCGG CCATCCGCTG 1680

CCGCTGCGCC TGGACGCCAT CACCGGCCCC GAGGAGGAAG GCGGGCGCCT GGAGACCATT 1740

CTCGGCTGGC CGCTGGCCGA GCGCACCGTG GTGATTCCCT CGGCGATCCC CACCGACCCG 1800

CGCAACGTCG GCGGCGACCT CGACCCGTCC AGCATCCCCG ACAAGGAACA GGCGATCAGC 1860

GCCCTGCCGG ACTACGCCAG CCAGCCCGGC AAACCGCCGC GCGAGGACCC GCTAGCACCC 1920

GGGATCCCGT CAGGCCCCCT CAAAGCCGAG ATCGCACAGA GACTTGAAGA TGTCTTTGCA 1980

GGGAAGAACA CCGATCTTGA GGTTCTCATG GAATGGCTAA AGACAAGACC AATCCTGTCA 2040

CCTCTGACTA AGGGGATTTT AGGATTTGTG TTCACGCTCA CCGTGCCCAG TGAGCGAGGA 2100

CTGCAGCGTA GACGCTTTGT CCAAAATGCC CTTAATGGGA ACGGGGATCC AAATAACATG 2160

GACAAAGCAG TTAAACTGTA TAGGAAGCTC AAGAGGGAGC CCGGGAAACC GCCGCGCGAG 2220

GACCTGAAGT AAGAATTC 2238

(2) INFORMATION FOR SEQ ID NO:29:

(i) SEQUENCE CHARACTERISTICS:
(A) LENGTH: 746 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:29:

Met His Leu Ile Pro His Trp Ile Pro Leu Val Ala Ser Leu Gly Leu
1               5                   10                  15

Leu Ala Gly Gly Ser Ser Ala Ser Ala Ala Glu Glu Ala Phe Asp Leu
            20                  25                  30

Trp Asn Glu Cys Ala Lys Ala Cys Val Leu Asp Leu Lys Asp Gly Val
        35                  40                  45

Arg Ser Ser Arg Met Ser Val Asp Pro Ala Ile Ala Asp Thr Asn Gly
    50                  55                  60

Gln Gly Val Leu His Tyr Ser Met Val Leu Glu Gly Gly Asn Asp Ala
65                  70                  75                  80

Leu Lys Leu Ala Ile Asp Asn Ala Leu Ser Ile Thr Ser Asp Gly Leu
                85                  90                  95

Thr Ile Arg Leu Glu Gly Gly Val Glu Pro Asn Lys Pro Val Arg Tyr
            100                 105                 110

Ser Tyr Thr Arg Gln Ala Arg Gly Ser Trp Ser Leu Asn Trp Leu Val
        115                 120                 125

Pro Ile Gly His Glu Lys Pro Ser Asn Ile Lys Val Phe Ile His Glu
    130                 135                 140

Leu Asn Ala Gly Asn Gln Leu Ser His Met Ser Pro Ile Tyr Thr Ile
145                 150                 155                 160

Glu Met Gly Asp Glu Leu Leu Ala Lys Leu Ala Arg Asp Ala Thr Phe
                165                 170                 175

Phe Val Arg Ala His Glu Ser Asn Glu Met Gln Pro Thr Leu Ala Ile
            180                 185                 190

Ser His Ala Gly Val Ser Val Val Met Ala Gln Thr Gln Pro Arg Arg
        195                 200                 205

Glu Lys Arg Trp Ser Glu Trp Ala Ser Gly Lys Val Leu Cys Leu Leu
    210                 215                 220

Asp Pro Leu Asp Gly Val Tyr Asn Tyr Leu Ala Gln Gln Arg Cys Asn
225                 230                 235                 240

Leu Asp Asp Thr Trp Glu Gly Lys Ile Tyr Arg Val Leu Ala Gly Asn
                245                 250                 255

Pro Ala Lys His Asp Leu Asp Ile Lys Pro Thr Val Ile Ser His Arg
260 265 270

Leu His Phe Pro Glu Gly Gly Ser Leu Ala Ala Leu Thr Ala His Gln
275 280 285

Ala Cys His Leu Pro Leu Glu Thr Phe Thr Arg His Arg Gln Pro Arg
290 295 300

Gly Trp Glu Gln Leu Glu Gln Cys Gly Tyr Pro Val Gln Arg Leu Val
305 310 315 320

Ala Leu Tyr Leu Ala Ala Arg Leu Ser Trp Asn Gln Val Asp Gln Val
325 330 335

Ile Arg Asn Ala Leu Ala Ser Pro Gly Ser Gly Gly Asp Leu Gly Glu
340 345 350

Ala Ile Arg Glu Gln Pro Glu Gln Ala Arg Leu Ala Leu Thr Leu Ala
355 360 365

Ala Ala Glu Ser Glu Arg Phe Val Arg Gln Gly Thr Gly Asn Asp Glu
370 375 380

Ala Gly Ala Ala Asn Ala Asp Val Val Ser Leu Thr Cys Pro Val Ala
385 390 395 400

Ala Gly Glu Cys Ala Gly Pro Ala Asp Ser Gly Asp Ala Leu Leu Glu
405 410 415

Arg Asn Tyr Pro Thr Gly Ala Glu Phe Leu Gly Asp Gly Gly Asp Val
420 425 430

Ser Phe Ser Thr Arg Gly Thr Gln Asn Trp Thr Val Glu Arg Leu Leu
435 440 445

Gln Ala His Arg Gln Leu Glu Glu Arg Gly Tyr Val Phe Val Gly Tyr
450 455 460

His Gly Thr Phe Leu Glu Ala Ala Gln Ser Ile Val Phe Gly Gly Val
465 470 475 480

Arg Ala Arg Ser Gln Asp Leu Asp Ala Ile Trp Arg Gly Phe Tyr Ile
485 490 495

Ala Gly Asp Pro Ala Leu Ala Tyr Gly Tyr Ala Gln Asp Gln Glu Pro
500 505 510

Asp Ala Arg Gly Arg Ile Arg Asn Gly Ala Leu Leu Arg Val Tyr Val
515 520 525

Pro Arg Ser Ser Leu Pro Gly Phe Tyr Arg Thr Ser Leu Thr Leu Ala
530 535 540

Ala Pro Glu Ala Ala Gly Glu Val Glu Arg Leu Ile Gly His Pro Leu
545 550 555 560

Pro Leu Arg Leu Asp Ala Ile Thr Gly Pro Glu Glu Glu Gly Gly Arg
565 570 575

Leu Glu Thr Ile Leu Gly Trp Pro Leu Ala Glu Arg Thr Val Val Ile
580 585 590

Pro Ser Ala Ile Pro Thr Asp Pro Arg Asn Val Gly Gly Asp Leu Asp
595 600 605

Pro Ser Ser Ile Pro Asp Lys Glu Gln Ala Ile Ser Ala Leu Pro Asp
610 615 620

Tyr Ala Ser Gln Pro Gly Lys Pro Pro Arg Glu Asp Pro Leu Ala Pro
625 630 635 640

Gly Ile Pro Ser Gly Pro Leu Lys Ala Glu Ile Ala Gln Arg Leu Glu
645 650 655

Asp Val Phe Ala Gly Lys Asn Thr Asp Leu Glu Val Leu Met Glu Trp
660 665 670

Leu Lys Thr Arg Pro Ile Leu Ser Pro Leu Thr Lys Gly Ile Leu Gly
675 680 685

Phe Val Phe Thr Leu Thr Val Pro Ser Glu Arg Gly Leu Gln Arg Arg
690 695 700

Arg Phe Val Gln Asn Ala Leu Asn Gly Asn Gly Asp Pro Asn Asn Met
705 710 715 720

Asp Lys Ala Val Lys Leu Tyr Arg Lys Leu Lys Arg Glu Pro Gly Lys
725 730 735

Pro Pro Arg Glu Asp Leu Lys Xaa Glu Phe
740 745

(2) INFORMATION FOR SEQ ID NO:30:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:30:

CTAGACTAGT CTAG 14

(2) INFORMATION FOR SEQ ID NO:31:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:31:

GGCGGCAGAA AGAGC 15

(2) INFORMATION FOR SEQ ID NO:32:

(i) SEQUENCE CHARACTERISTICS:
(A) LENGTH: 21 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:32:

```
Met Lys Ala Asn Leu Leu Val Leu Leu Cys Ala Leu Ala Ala Ala Asp
1               5                   10                  15
```

Ala Asp Thr Ile Cys
20

(2) INFORMATION FOR SEQ ID NO:33:

(i) SEQUENCE CHARACTERISTICS:
(A) LENGTH: 72 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:33:

GGCAGAAAGA TGAAGGCAAA CCTACTGGTC CTGTTATGTG CACTTGCAGC TGCAGATGCA 60

GACACAATAT GC 72

(2) INFORMATION FOR SEQ ID NO:34:

(i) SEQUENCE CHARACTERISTICS:
(A) LENGTH: 24 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:34:

Gly Arg Lys Met Lys Ala Asn Leu Leu Val Leu Leu Cys Ala Leu Ala
1 5 10 15

Ala Ala Asp Ala Asp Thr Ile Cys
20

(2) INFORMATION FOR SEQ ID NO:35:

(i) SEQUENCE CHARACTERISTICS:
(A) LENGTH: 63 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:35:

```
ATGAAGGCAA ACCTACTGGT CCTGTTATGT GCACTTGCAG CTGCAGATGC AGACACAATA        60

TGA                                                                      63
```

(2) INFORMATION FOR SEQ ID NO:36:

(i) SEQUENCE CHARACTERISTICS:
(A) LENGTH: 21 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:36:

```
Met Lys Ala Asn Leu Leu Val Leu Leu Cys Ala Leu Ala Ala Ala Asp
1               5                   10                  15

Ala Asp Thr Ile Xaa
            20
```

(2) INFORMATION FOR SEQ ID NO:37:

(i) SEQUENCE CHARACTERISTICS:
(A) LENGTH: 27 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:37:

```
His His Ala Asn Glu Asn Ile Phe Tyr Cys Pro Ile Ala Ile Met Ser
1               5                   10                  15

Ala Leu Ala Met Val Tyr Leu Gly Ala Lys Asp
            20                  25
```

(2) INFORMATION FOR SEQ ID NO:38:

(i) SEQUENCE CHARACTERISTICS:
(A) LENGTH: 81 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:38:

CACCATGCCA ATGAGAACAT CTTCTACTGC CCCATTGCCA TCATGTCAGC TCTAGCCATG 60

GTATACCTGG GTGCAAAAAG C 81

(2) INFORMATION FOR SEQ ID NO:39:

(i) SEQUENCE CHARACTERISTICS:
(A) LENGTH: 27 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:39:

His His Ala Asn Glu Asn Ile Phe Tyr Cys Pro Ile Ala Ile Met Ser
1 5 10 15

Ala Leu Ala Met Val Tyr Leu Gly Ala Lys Ser
20 25

(2) INFORMATION FOR SEQ ID NO:40:

(i) SEQUENCE CHARACTERISTICS:
(A) LENGTH: 78 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:40:

GGCAGAAAGA TGAAGGCAAA CCTACTGGTC CTGTTATGTG CACTTGCAGC TGCAGATGCA 60

GACACAATAT GCATGATG 78

(2) INFORMATION FOR SEQ ID NO:41:

(i) SEQUENCE CHARACTERISTICS:
(A) LENGTH: 26 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:41:

Gly Arg Lys Met Lys Ala Asn Leu Leu Val Leu Leu Cys Ala Leu Ala
1 5 10 15

Ala Ala Asp Ala Asp Thr Ile Cys Met Met
20 25

(2) INFORMATION FOR SEQ ID NO:42:

(i) SEQUENCE CHARACTERISTICS:
(A) LENGTH: 72 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:42:

GGCATGAAGG CAAACCTACT GGTCCTGTTA TGTGCACTTG CAGCTGCAGA TGCAGACACA 60

ATATGCATGA TG 72

(2) INFORMATION FOR SEQ ID NO:43:

(i) SEQUENCE CHARACTERISTICS:
(A) LENGTH: 24 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:43:

```
Gly Met Lys Ala Asn Leu Leu Val Leu Leu Cys Ala Leu Ala Ala Ala
1               5                   10                  15

Asp Ala Asp Thr Ile Cys Met Met
            20
```

(2) INFORMATION FOR SEQ ID NO:44:

(i) SEQUENCE CHARACTERISTICS:
(A) LENGTH: 90 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:44:

```
GTATGCATGC ACCATGCCAA TGAGAACATC TTCTACTGCC CCATTGCCAT CATGTCAGCT        60

CTAGCCATGG TATACCTGGG TGCAAAAGAC                                         90
```

(2) INFORMATION FOR SEQ ID NO:45:

(i) SEQUENCE CHARACTERISTICS:
(A) LENGTH: 30 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:45:

```
Val Cys Met His His Ala Asn Glu Asn Ile Phe Tyr Cys Pro Ile Ala
1               5                   10                  15

Ile Met Ser Ala Leu Ala Met Val Tyr Leu Gly Ala Lys Asp
            20                  25                  30
```

(2) INFORMATION FOR SEQ ID NO:46:

(i) SEQUENCE CHARACTERISTICS:
(A) LENGTH: 147 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:46:

```
ATGAAGGCAA ACCTACTGGT CCTGTTATGT GCACTTGCAG CTGCAGATGC AGACACAATA    60

TGCCACCATG CCAATGAGAA CATCTTCTAC TGCCCCATTG CCATCATGTC AGCTCTAGCC   120

ATGGTATACC TGGGTGCAAA AGACAGC                                       147
```

(2) INFORMATION FOR SEQ ID NO:47:

(i) SEQUENCE CHARACTERISTICS:
(A) LENGTH: 49 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:47:

```
Met Lys Ala Asn Leu Leu Val Leu Leu Cys Ala Leu Ala Ala Ala Asp
1               5                   10                  15

Ala Asp Thr Ile Cys His His Ala Asn Glu Asn Ile Phe Tyr Cys Pro
            20                  25                  30

Ile Ala Ile Met Ser Ala Leu Ala Met Val Tyr Leu Gly Ala Lys Asp
        35                  40                  45

Ser
```

(2) INFORMATION FOR SEQ ID NO:48:

(i) SEQUENCE CHARACTERISTICS:
(A) LENGTH: 70 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:48:

```
CCTATCAGAA ACGAATGGGG GTGCAGATGC AACGGTTCAA GCGCGAGGAC CTGAAGTAAG        60

AATTCGAGCT                                                               70
```

(2) INFORMATION FOR SEQ ID NO:49:

(i) SEQUENCE CHARACTERISTICS:
(A) LENGTH: 2013 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:49:

```
ATGGCCGAGG AAGCTTTCGA CCTCTGGAAC GAATGCGCCA AAGCCTGCGT GCTCGACCTC    60
AAGGACGGCG TGCGTTCCAG CCGCATGAGC GTCGACCCGG CCATCGCCGA CACCAACGGC   120
CAGGGCGTGC TGCACTACTC CATGGTCCTG GAGGGCGGCA ACGACGCGCT CAAGCTGGCC   180
ATCGACAACG CCCTCAGCAT CACCAGCGAC GGCCTGACCA TCCGCCTCGA AGGCGGCGTC   240
GAGCCGAACA AGCCGGTGCG CTACAGCTAC ACGCGCCAGG CGCGCGGCAG TTGGTCGCTG   300
AACTGGCTGG TACCGATCGG CCACGAGAAG CCCTCGAACA TCAAGGTGTT CATCCACGAA   360
CTGAACGCCG GCAACCAGCT CAGCCACATG TCGCCGATCT ACACCATCGA GATGGGCGAC   420
GAGTTGCTGG CGAAGCTGGC GCGCGATGCC ACCTTCTTCG TCAGGGCGCA CGAGAGCAAC   480
GAGATGCAGC CGACGCTCGC CATCAGCCAT GCCGGGGTCA GCGTGGTCAT GGCCCAGACC   540
CAGCCGCGCC GGGAAAAGCG CTGGAGCGAA TGGGCCAGCG GCAAGGTGTT GTGCCTGCTC   600
GACCCGCTGG ACGGGGTCTA CAACTACCTC GCCCAGCAAC GCTGCAACCT CGACGATACC   660
TGGGAAGGCA AGATCTACCG GGTGCTCGCC GGCAACCCGG CGAAGCATGA CCTGGACATC   720
AAACCCACGG TCATCAGTCA TCGCCTGCAC TTTCCCGAGG GCGGCAGCCT GGCCGCGCTG   780
ACCGCGCACC AGGCTTGCCA CCTGCCGCTG GAGACTTTCA CCCGTCATCG CCAGCCGCGC   840
GGCTGGGAAC AACTGGAGCA GTGCGGCTAT CCGGTGCAGC GGCTGGTCGC CCTCTACCTG   900
GCGGCGCGGC TGTCGTGGAA CCAGGTCGAC CAGGTGATCC GCAACGCCCT GGCCAGCCCC   960
GGCAGCGGCG GCGACCTGGG CGAAGCGATC CGCGAGCAGC CGGAGCAGGC CCGTCTGGCC  1020
CTGACCCTGG CCGCCGCCGA GAGCGAGCGC TTCGTCCGGC AGGGCACCGG CAACGACGAG  1080
GCCGGCGCGG CCAACGCCGA CGTGGTGAGC CTGACCTGCC CGGTCGCCGC CGGTGAATGC  1140
GCGGGCCCGG CGGACAGCGG CGACGCCCTG CTGGAGCGCA ACTATCCCAC TGGCGCGGAG  1200
TTCCTCGGCG ACGGCGGCGA CGTCAGCTTC AGCACCCGCG GCAGTCTTCT AACCGAGGTC  1260
GAAACGTACG TTCTCTCTAT CATCCCGTCA GGCCCCCTCA AAGCCGAGAT CGCACAGAGA  1320
CTTGAAGATG TCTTTGCAGG GAAGAACACC GATCTTGAGG TTCTCATGGA ATGGCTAAAG  1380
```

ACAAGACCAA TCCTGTCACC TCTGACTAAG GGGATTTTAG GATTTGTGTT CACGCTCACC 1440

GTGCCCAGTG AGCGAGGACT GCAGCGTAGA CGCTTTGTCC AAAATGCCCT TAATGGGAAC 1500

GGGGATCCAA ATAACATGGA CAAAGCAGTT AAACTGTATA GGAAGCTCAA GAGGGAGATA 1560

ACATTCCATG GGGCCAAAGA AATCTCACTC AGTTATTCTG CTGGTGCACT TGCCAGTTGT 1620

ATGGGCCTCA TATACAACAG GATGGGGGCT GTGACCACTG AAGTGGCATT TGGCCTGGTA 1680

TGTGCAACCT GTGAACAGAT TGCTGACTCC CAGCATCGGT CTCATAGGCA AATGGTGACA 1740

ACAACCAACC CACTAATCAG ACATGAGAAC AGAATGGTTT TAGCCAGCAC TACAGCTAAG 1800

GCTATGGAGC AAATGGCTGG ATCGAGTGAG CAAGCAGCAG AGGCCATGGA GGTTGCTAGT 1860

CAGGCTAGGC AAATGGTGCA AGCGATGAGA ACCATTGGGA CTCATCCTAG CTCCAGTGCT 1920

GGTCTGAAAA ATGATCTTCT TGAAAATTTG CAGGCCTATC AGAAACGAAT GGGGGTGCAG 1980

ATGCAACGGT TCAAGCGCGA GGACCTGAAG TAA 2013

(2) INFORMATION FOR SEQ ID NO:50:

(i) SEQUENCE CHARACTERISTICS:
(A) LENGTH: 671 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:50:

Met Ala Glu Glu Ala Phe Asp Leu Trp Asn Glu Cys Ala Lys Ala Cys
1 5 10 15

Val Leu Asp Leu Lys Asp Gly Val Arg Ser Ser Arg Met Ser Val Asp
20 25 30

Pro Ala Ile Ala Asp Thr Asn Gly Gln Gly Val Leu His Tyr Ser Met
35 40 45

Val Leu Glu Gly Gly Asn Asp Ala Leu Lys Leu Ala Ile Asp Asn Ala
50 55 60

Leu Ser Ile Thr Ser Asp Gly Leu Thr Ile Arg Leu Glu Gly Gly Val
65 70 75 80

Glu Pro Asn Lys Pro Val Arg Tyr Ser Tyr Thr Arg Gln Ala Arg Gly
85 90 95

Ser Trp Ser Leu Asn Trp Leu Val Pro Ile Gly His Glu Lys Pro Ser
100 105 110

Asn Ile Lys Val Phe Ile His Glu Leu Asn Ala Gly Asn Gln Leu Ser
115 120 125

His Met Ser Pro Ile Tyr Thr Ile Glu Met Gly Asp Glu Leu Leu Ala
130 135 140

Lys Leu Ala Arg Asp Ala Thr Phe Phe Val Arg Ala His Glu Ser Asn
145 150 155 160

Glu Met Gln Pro Thr Leu Ala Ile Ser His Ala Gly Val Ser Val Val
165 170 175

Met Ala Gln Thr Gln Pro Arg Arg Glu Lys Arg Trp Ser Glu Trp Ala
180 185 190

Ser Gly Lys Val Leu Cys Leu Leu Asp Pro Leu Asp Gly Val Tyr Asn
195 200 205

Tyr Leu Ala Gln Gln Arg Cys Asn Leu Asp Asp Thr Trp Glu Gly Lys
210 215 220

Ile Tyr Arg Val Leu Ala Gly Asn Pro Ala Lys His Asp Leu Asp Ile
225 230 235 240

Lys Pro Thr Val Ile Ser His Arg Leu His Phe Pro Glu Gly Gly Ser
245 250 255

Leu Ala Ala Leu Thr Ala His Gln Ala Cys His Leu Pro Leu Glu Thr
260 265 270

Phe Thr Arg His Arg Gln Pro Arg Gly Trp Glu Gln Leu Glu Gln Cys
275 280 285

Gly Tyr Pro Val Gln Arg Leu Val Ala Leu Tyr Leu Ala Ala Arg Leu
290 295 300

Ser Trp Asn Gln Val Asp Gln Val Ile Arg Asn Ala Leu Ala Ser Pro
305 310 315 320

Gly Ser Gly Gly Asp Leu Gly Glu Ala Ile Arg Glu Gln Pro Glu Gln
325 330 335

Ala Arg Leu Ala Leu Thr Leu Ala Ala Ala Glu Ser Glu Arg Phe Val
340 345 350

Arg Gln Gly Thr Gly Asn Asp Glu Ala Gly Ala Ala Asn Ala Asp Val
355 360 365

Val Ser Leu Thr Cys Pro Val Ala Ala Gly Glu Cys Ala Gly Pro Ala
370 375 380

Asp Ser Gly Asp Ala Leu Leu Glu Arg Asn Tyr Pro Thr Gly Ala Glu
385 390 395 400

Phe Leu Gly Asp Gly Gly Asp Val Ser Phe Ser Thr Arg Gly Ser Leu
405 410 415

Leu Thr Glu Val Glu Thr Tyr Val Leu Ser Ile Ile Pro Ser Gly Pro
420 425 430

Leu Lys Ala Glu Ile Ala Gln Arg Leu Glu Asp Val Phe Ala Gly Lys
435 440 445

Asn Thr Asp Leu Glu Val Leu Met Glu Trp Leu Lys Thr Arg Pro Ile
450 455 460

Leu Ser Pro Leu Thr Lys Gly Ile Leu Gly Phe Val Phe Thr Leu Thr
465 470 475 480

Val Pro Ser Glu Arg Gly Leu Gln Arg Arg Arg Phe Val Gln Asn Ala
485 490 495

Leu Asn Gly Asn Gly Asp Pro Asn Asn Met Asp Lys Ala Val Lys Leu
500 505 510

Tyr Arg Lys Leu Lys Arg Glu Ile Thr Phe His Gly Ala Lys Glu Ile
515 520 525

Ser Leu Ser Tyr Ser Ala Gly Ala Leu Ala Ser Cys Met Gly Leu Ile
530 535 540

Tyr Asn Arg Met Gly Ala Val Thr Thr Glu Val Ala Phe Gly Leu Val
545 550 555 560

Cys Ala Thr Cys Glu Gln Ile Ala Asp Ser Gln His Arg Ser His Arg
565 570 575

```
Gln Met Val Thr Thr Thr Asn Pro Leu Ile Arg His Glu Asn Arg Met
            580                 585                 590

Val Leu Ala Ser Thr Thr Ala Lys Ala Met Glu Gln Met Ala Gly Ser
        595                 600                 605

Ser Glu Gln Ala Ala Glu Ala Met Glu Val Ala Ser Gln Ala Arg Gln
    610                 615                 620

Met Val Gln Ala Met Arg Thr Ile Gly Thr His Pro Ser Ser Ser Ala
625                 630                 635                 640

Gly Leu Lys Asn Asp Leu Leu Glu Asn Leu Gln Ala Tyr Gln Lys Arg
                645                 650                 655

Met Gly Val Gln Met Gln Arg Phe Lys Arg Glu Asp Leu Lys Xaa
            660                 665                 670
```

(2) INFORMATION FOR SEQ ID NO:51:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:51:

```
ATACCCGCGG CATGGCGTCC CAAGGCACCA AACGGTCT                          38
```

(2) INFORMATION FOR SEQ ID NO:52:

(i) SEQUENCE CHARACTERISTICS:
(A) LENGTH: 81 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:52:

ATAGAATTCT TACTTCAGGT CCTCGCGATT GTCGTACTCC TCTGCATTGT CTCCGAAGAA 60

ATAAGATCCT TCATTACTCA T 81

(2) INFORMATION FOR SEQ ID NO:53:

(i) SEQUENCE CHARACTERISTICS:
(A) LENGTH: 2754 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:53:

ATGGCCGAGG AAGCTTTCGA CCTCTGGAAC GAATGCGCCA AAGCCTGCGT GCTCGACCTC 60

AAGGACGGCG TGCGTTCCAG CCGCATGAGC GTCGACCCGG CCATCGCCGA CACCAACGGC 120

CAGGGCGTGC TGCACTACTC CATGGTCCTG GAGGGCGGCA ACGACGCGCT CAAGCTGGCC 180

ATCGACAACG CCCTCAGCAT CACCAGCGAC GGCCTGACCA TCCGCCTCGA AGGCGGCGTC 240

GAGCCGAACA AGCCGGTGCG CTACAGCTAC ACGCGCCAGG CGCGCGGCAG TTGGTCGCTG 300

AACTGGCTGG TACCGATCGG CCACGAGAAG CCCTCGAACA TCAAGGTGTT CATCCACGAA 360

CTGAACGCCG GCAACCAGCT CAGCCACATG TCGCCGATCT ACACCATCGA GATGGGCGAC 420

GAGTTGCTGG CGAAGCTGGC GCGCGATGCC ACCTTCTTCG TCAGGGCGCA CGAGAGCAAC 480

GAGATGCAGC CGACGCTCGC CATCAGCCAT GCCGGGGTCA GCGTGGTCAT GGCCCAGACC 540

CAGCCGCGCC GGGAAAAGCG CTGGAGCGAA TGGGCCAGCG GCAAGGTGTT GTGCCTGCTC 600

GACCCGCTGG ACGGGGTCTA CAACTACCTC GCCCAGCAAC GCTGCAACCT CGACGATACC 660

TGGGAAGGCA AGATCTACCG GGTGCTCGCC GGCAACCCGG CGAAGCATGA CCTGGACATC 720

AAACCCACGG TCATCAGTCA TCGCCTGCAC TTTCCCGAGG GCGGCAGCCT GGCCGCGCTG 780

ACCGCGCACC AGGCTTGCCA CCTGCCGCTG GAGACTTTCA CCCGTCATCG CCAGCCGCGC 840

GGCTGGGAAC AACTGGAGCA GTGCGGCTAT CCGGTGCAGC GGCTGGTCGC CCTCTACCTG 900

GCGGCGCGGC TGTCGTGGAA CCAGGTCGAC CAGGTGATCC GCAACGCCCT GGCCAGCCCC 960

GGCAGCGGCG GCGACCTGGG CGAAGCGATC CGCGAGCAGC CGGAGCAGGC CCGTCTGGCC 1020

CTGACCCTGG CCGCCGCCGA GAGCGAGCGC TTCGTCCGGC AGGGCACCGG CAACGACGAG 1080

GCCGGCGCGG CCAACGCCGA CGTGGTGAGC CTGACCTGCC CGGTCGCCGC CGGTGAATGC 1140

GCGGGCCCGG CGGACAGCGG CGACGCCCTG CTGGAGCGCA ACTATCCCAC TGGCGCGGAG 1200

TTCCTCGGCG ACGGCGGCGA CGTCAGCTTC AGCACCCGCG GCATGGCGTC CCAAGGCACC 1260

AAACGGTCTT ACGAACAGAT GGAGACTGAT GGAGAACGCC AGAATGCCAC TGAAATCAGA 1320

GCATCCGTCG GAAAAATGAT TGGTGGAATT GGACGATTCT ACATCCAAAT GTGCACAGAA 1380

CTTAAACTCA GTGATTATGA GGGACGGTTG ATCCAAAACA GCTTAACAAT AGAGAGAATG 1440

GTGCTCTCTG CTTTTGACGA AAGGAGAAAT AAATACCTGG AAGAACATCC CAGTGCGGGG 1500

AAGGATCCTA AGAAAACTGG AGGACCTATA TACAGAAGAG TAAACGGAAA GTGGATGAGA 1560

GAACTCATCC TTTATGACAA AGAAGAAATA AGGCGAATCT GGCGCCAAGC TAATAATGGT 1620

GACGATGCAA CGGCTGGTCT GACTCACATG ATGATCTGGC ATTCCAATTT GAATGATGCA 1680

ACTTATCAGA GGACAAGGGC TCTTGTTCGC ACCGGAATGG ATCCCAGGAT GTGCTCTCTG 1740

ATGCAAGGTT CAACTCTCCC TAGGAGGTCT GGAGCCGCAG GTGCTGCAGT CAAAGGAGTT 1800

GGAACAATGG TGATGGAATT GGTCAGGATG ATCAAACGTG GGATCAATGA TCGGAACTTC 1860

TGGAGGGGTG AGAATGGACG AAAAACAAGA ATTGCTTATG AAAGAATGTG CAACATTCTC 1920

AAAGGGAAAT TTCAAACTGC TGCACAAAAA GCAATGATGG ATCAAGTGAG AGAGAGCCGG 1980

GACCCAGGGA ATGCTGAGTT CGAAGATCTC ACTTTTCTAG CACGGTCTGC ACTCATATTG 2040

AGAGGGTCGG TTGCTCACAA GTCCTGCCTG CCTGCCTGTG TGTATGGACC TGCCGTAGCC 2100

AGTGGGTACG ACTTTGAAAG AGAGGGATAC TCTCTAGTCG GAATAGACCC TTTCAGACTG 2160

CTTCAAAACA GCCAAGTGTA CAGCCTAATC AGACCAAATG AGAATCCAGC ACACAAGAGT 2220

CAACTGGTGT GGATGGCATG CCATTCTGCC GCATTTGAAG ATCTAAGAGT ATTGAGCTTC 2280

ATCAAAGGGA CGAAGGTGGT CCCAAGAGGG AAGCTTTCCA CTAGAGGAGT TCAAATTGCT 2340

TCCAATGAAA ATATGGAGAC TATGGAATCA AGTACACTTG AACTGAGAAG CAGGTACTGG 2400

GCCATAAGGA CCAGAAGTGG AGGAAACACC AATCAACAGA GGGCATCTGC GGGCCAAATC 2460

AGCATACAAC CTACGTTCTC AGTACAGAGA AATCTCCCTT TTGACAGAAC AACCGTTATG 2520

GCAGCATTCA CTGGGAATAC AGAGGGGAGA ACATCTGACA TGAGGACCGA AATCATAAGG 2580

ATGATGGAAA GTGCAAGACC AGAAGATGTG TCTTTCCAGG GGCGGGGAGT CTTCGAGCTC 2640

TCGGACGAAA AGGCAGCGAG CCCGATCGTG CCTTCCTTTG ACATGAGTAA TGAAGGATCT 2700

TATTTCTTCG GAGACAATGC AGAGGAGTAC GACAATCGCG AGGACCTGAA GTAA 2754

(2) INFORMATION FOR SEQ ID NO:54:

(i) SEQUENCE CHARACTERISTICS:
(A) LENGTH: 918 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:54:

Met Ala Glu Glu Ala Phe Asp Leu Trp Asn Glu Cys Ala Lys Ala Cys
1 5 10 15

Val Leu Asp Leu Lys Asp Gly Val Arg Ser Ser Arg Met Ser Val Asp
20 25 30

Pro Ala Ile Ala Asp Thr Asn Gly Gln Gly Val Leu His Tyr Ser Met
35 40 45

Val Leu Glu Gly Gly Asn Asp Ala Leu Lys Leu Ala Ile Asp Asn Ala
50 55 60

Leu Ser Ile Thr Ser Asp Gly Leu Thr Ile Arg Leu Glu Gly Gly Val
65 70 75 80

Glu Pro Asn Lys Pro Val Arg Tyr Ser Tyr Thr Arg Gln Ala Arg Gly
85 90 95

Ser Trp Ser Leu Asn Trp Leu Val Pro Ile Gly His Glu Lys Pro Ser
100 105 110

Asn Ile Lys Val Phe Ile His Glu Leu Asn Ala Gly Asn Gln Leu Ser
115 120 125

His Met Ser Pro Ile Tyr Thr Ile Glu Met Gly Asp Glu Leu Leu Ala
130 135 140

Lys Leu Ala Arg Asp Ala Thr Phe Phe Val Arg Ala His Glu Ser Asn
145 150 155 160

Glu Met Gln Pro Thr Leu Ala Ile Ser His Ala Gly Val Ser Val Val
165 170 175

Met Ala Gln Thr Gln Pro Arg Arg Glu Lys Arg Trp Ser Glu Trp Ala
180 185 190

Ser Gly Lys Val Leu Cys Leu Leu Asp Pro Leu Asp Gly Val Tyr Asn
195 200 205

Tyr Leu Ala Gln Gln Arg Cys Asn Leu Asp Asp Thr Trp Glu Gly Lys
210 215 220

Ile Tyr Arg Val Leu Ala Gly Asn Pro Ala Lys His Asp Leu Asp Ile
225 230 235 240

Lys Pro Thr Val Ile Ser His Arg Leu His Phe Pro Glu Gly Gly Ser
245 250 255

Leu Ala Ala Leu Thr Ala His Gln Ala Cys His Leu Pro Leu Glu Thr
260 265 270

Phe Thr Arg His Arg Gln Pro Arg Gly Trp Glu Gln Leu Glu Gln Cys
275 280 285

Gly Tyr Pro Val Gln Arg Leu Val Ala Leu Tyr Leu Ala Ala Arg Leu
290 295 300

Ser Trp Asn Gln Val Asp Gln Val Ile Arg Asn Ala Leu Ala Ser Pro
305 310 315 320

Gly Ser Gly Gly Asp Leu Gly Glu Ala Ile Arg Glu Gln Pro Glu Gln
325 330 335

Ala Arg Leu Ala Leu Thr Leu Ala Ala Ala Glu Ser Glu Arg Phe Val
340 345 350

Arg Gln Gly Thr Gly Asn Asp Glu Ala Gly Ala Ala Asn Ala Asp Val
355 360 365

Val Ser Leu Thr Cys Pro Val Ala Ala Gly Glu Cys Ala Gly Pro Ala
370 375 380

Asp Ser Gly Asp Ala Leu Leu Glu Arg Asn Tyr Pro Thr Gly Ala Glu
385 390 395 400

Phe Leu Gly Asp Gly Gly Asp Val Ser Phe Ser Thr Arg Gly Met Ala
405 410 415

Ser Gln Gly Thr Lys Arg Ser Tyr Glu Gln Met Glu Thr Asp Gly Glu
420 425 430

Arg Gln Asn Ala Thr Glu Ile Arg Ala Ser Val Gly Lys Met Ile Gly
435 440 445

Gly Ile Gly Arg Phe Tyr Ile Gln Met Cys Thr Glu Leu Lys Leu Ser
450 455 460

Asp Tyr Glu Gly Arg Leu Ile Gln Asn Ser Leu Thr Ile Glu Arg Met
465 470 475 480

Val Leu Ser Ala Phe Asp Glu Arg Arg Asn Lys Tyr Leu Glu Glu His
485 490 495

Pro Ser Ala Gly Lys Asp Pro Lys Lys Thr Gly Gly Pro Ile Tyr Arg
500 505 510

Arg Val Asn Gly Lys Trp Met Arg Glu Leu Ile Leu Tyr Asp Lys Glu
515 520 525

Glu Ile Arg Arg Ile Trp Arg Gln Ala Asn Asn Gly Asp Asp Ala Thr
530 535 540

Ala Gly Leu Thr His Met Met Ile Trp His Ser Asn Leu Asn Asp Ala
545 550 555 560

Thr Tyr Gln Arg Thr Arg Ala Leu Val Arg Thr Gly Met Asp Pro Arg
565 570 575

Met Cys Ser Leu Met Gln Gly Ser Thr Leu Pro Arg Arg Ser Gly Ala
580 585 590

Ala Gly Ala Ala Val Lys Gly Val Gly Thr Met Val Met Glu Leu Val
595 600 605

Arg Met Ile Lys Arg Gly Ile Asn Asp Arg Asn Phe Trp Arg Gly Glu
610 615 620

Asn Gly Arg Lys Thr Arg Ile Ala Tyr Glu Arg Met Cys Asn Ile Leu
625 630 635 640

Lys Gly Lys Phe Gln Thr Ala Ala Gln Lys Ala Met Met Asp Gln Val
645 650 655

Arg Glu Ser Arg Asp Pro Gly Asn Ala Glu Phe Glu Asp Leu Thr Phe
660 665 670

Leu Ala Arg Ser Ala Leu Ile Leu Arg Gly Ser Val Ala His Lys Ser
675 680 685

Cys Leu Pro Ala Cys Val Tyr Gly Pro Ala Val Ala Ser Gly Tyr Asp
690 695 700

Phe Glu Arg Glu Gly Tyr Ser Leu Val Gly Ile Asp Pro Phe Arg Leu
705 710 715 720

Leu Gln Asn Ser Gln Val Tyr Ser Leu Ile Arg Pro Asn Glu Asn Pro
725 730 735

Ala His Lys Ser Gln Leu Val Trp Met Ala Cys His Ser Ala Ala Phe
740 745 750

Glu Asp Leu Arg Val Leu Ser Phe Ile Lys Gly Thr Lys Val Val Pro
755 760 765

Arg Gly Lys Leu Ser Thr Arg Gly Val Gln Ile Ala Ser Asn Glu Asn
770 775 780

Met Glu Thr Met Glu Ser Ser Thr Leu Glu Leu Arg Ser Arg Tyr Trp
785 790 795 800

Ala Ile Arg Thr Arg Ser Gly Gly Asn Thr Asn Gln Gln Arg Ala Ser
805 810 815

Ala Gly Gln Ile Ser Ile Gln Pro Thr Phe Ser Val Gln Arg Asn Leu
820 825 830

Pro Phe Asp Arg Thr Thr Val Met Ala Ala Phe Thr Gly Asn Thr Glu
835 840 845

Gly Arg Thr Ser Asp Met Arg Thr Glu Ile Ile Arg Met Met Glu Ser
850 855 860

```
Ala Arg Pro Glu Asp Val Ser Phe Gln Gly Arg Gly Val Phe Glu Leu
865                 870                 875                 880

Ser Asp Glu Lys Ala Ala Ser Pro Ile Val Pro Ser Phe Asp Met Ser
                885                 890                 895

Asn Glu Gly Ser Tyr Phe Phe Gly Asp Asn Ala Glu Glu Tyr Asp Asn
            900                 905                 910

Arg Glu Asp Leu Lys Xaa
        915
```

(2) INFORMATION FOR SEQ ID NO:55:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:55:

ATACCCGCGG CATGGGTGCG AGAGCGTCGG TATAT 35

(2) INFORMATION FOR SEQ ID NO:56:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:56:

ATAGAATTCT CATTGTGACG AGGGGTCGCT GCCAAA 36

(2) INFORMATION FOR SEQ ID NO:57:

(i) SEQUENCE CHARACTERISTICS:
(A) LENGTH: 2814 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:57:

ATGAAAAAGA CAGCTATCGC GATTGCAGTG GCACTGGCTG GTTTCGCTAC CGTAGCGCAG 60

GCCGCGAATT TGGCCGAAGA AGCTTTCGAC CTCTGGAACG AATGCGCCAA AGCCTGCGTG 120

CTCGACCTCA AGGACGGCGT GCGTTCCAGC CGCATGAGCG TCGACCCGGC CATCGCCGAC 180

ACCAACGGCC AGGGCGTGCT GCACTACTCC ATGGTCCTGG AGGGCGGCAA CGACGCGCTC 240

AAGCTGGCCA TCGACAACGC CCTCAGCATC ACCAGCGACG GCCTGACCAT CCGCCTCGAA 300

GGCGGCGTCG AGCCGAACAA GCCGGTGCGC TACAGCTACA CGCGCCAGGC GCGCGGCAGT 360

TGGTCGCTGA ACTGGCTGGT ACCGATCGGC CACGAGAAGC CCTCGAACAT CAAGGTGTTC 420

ATCCACGAAC TGAACGCCGG CAACCAGCTC AGCCACATGT CGCCGATCTA CACCATCGAG 480

ATGGGCGACG AGTTGCTGGC GAAGCTGGCG CGCGATGCCA CCTTCTTCGT CAGGGCGCAC 540

GAGAGCAACG AGATGCAGCC GACGCTCGCC ATCAGCCATG CCGGGGTCAG CGTGGTCATG 600

GCCCAGACCC AGCCGCGCCG GGAAAAGCGC TGGAGCGAAT GGGCCAGCGG CAAGGTGTTG 660

TGCCTGCTCG ACCCGCTGGA CGGGGTCTAC AACTACCTCG CCCAGCAACG CTGCAACCTC 720

GACGATACCT GGGAAGGCAA GATCTACCGG GTGCTCGCCG GCAACCCGGC GAAGCATGAC 780

CTGGACATCA AACCCACGGT CATCAGTCAT CGCCTGCACT TTCCCGAGGG CGGCAGCCTG 840

GCCGCGCTGA CCGCGCACCA GGCTTGCCAC CTGCCGCTGG AGACTTTCAC CCGTCATCGC 900

CAGCCGCGCG GCTGGGAACA ACTGGAGCAG TGCGGCTATC CGGTGCAGCG GCTGGTCGCC 960

CTCTACCTGG CGGCGCGGCT GTCGTGGAAC CAGGTCGACC AGGTGATCCG CAACGCCCTG 1020

GCCAGCCCCG GCAGCGGCGG CGACCTGGGC GAAGCGATCC GCGAGCAGCC GGAGCAGGCC 1080
CGTCTGGCCC TGACCCTGGC CGCCGCCGAG AGCGAGCGCT TCGTCCGGCA GGGCACCGGC 1140
AACGACGAGG CCGGCGCGGC CAACGCCGAC GTGGTGAGCC TGACCTGCCC GGTCGCCGCC 1200
GGTGAATGCG CGGGCCCGGC GGACAGCGGC GACGCCCTGC TGGAGCGCAA CTATCCCACT 1260
GGCGCGGAGT TCCTCGGCGA CGGCGGCGAC GTCAGCTTCA GCACCCGCGG CATGGGTGCG 1320
AGAGCGTCGG TATTAAGCGG GGGAGAATTA GATAAATGGG AAAAAATTCG GTTAAGGCCA 1380
GGGGGAAAGA AACAATATAA ACTAAAACAT ATAGTATGGG CAAGCAGGGA GCTAGAACGA 1440
TTCGCAGTTA ATCCTGGCCT TTTAGAGACA TCAGAAGGCT GTAGACAAAT ACTGGGACAG 1500
CTACAACCAT CCCTTCAGAC AGGATCAGAA GAACTTAGAT CATTATATAA TACAATAGCA 1560
GTCCTCTATT GTGTGCATCA AAGGATAGAT GTAAAAGACA CCAAGGAAGC CTTAGATAAG 1620
ATAGAGGAAG AGCAAAACAA AAGTAAGAAA AAGGCACAGC AAGCAGCAGC TGACACAGGA 1680
AACAACAGCC AGGTCAGCCA AAATTACCCT ATAGTGCAGA ACCTCCAGGG GCAAATGGTA 1740
CATCAGGCCA TATCACCTAG AACTTTAAAT GCATGGGTAA AAGTAGTAGA AGAGAAGGCT 1800
TTCAGCCCAG AAGTAATACC CATGTTTTCA GCATTATCAG AAGGAGCCAC CCCACAAGAT 1860
TTAAATACCA TGCTAAACAC AGTGGGGGGA CATCAAGCAG CCATGCAAAT GTTAAAAGAG 1920
ACCATCAATG AGGAAGCTGC AGAATGGGAT AGATTGCATC CAGTGCATGC AGGGCCTATT 1980
GCACCAGGCC AGATGAGAGA ACCAAGGGGA AGTGACATAG CAGGAACTAC TAGTACCCTT 2040
CAGGAACAAA TAGGATGGAT GACACATAAT CCACCTATCC CAGTAGGAGA AATCTATAAA 2100
AGATGGATAA TCCTGGGATT AAATAAAATA GTAAGAATGT ATAGCCCTAC CAGCATTCTG 2160
GACATAAGAC AAGGACCAAA GGAACCCTTT AGAGACTATG TAGACCGATT CTATAAAACT 2220
CTAAGAGCCG AGCAAGCTTC ACAAGAGGTA AAAAATTGGA TGACAGAAAC CTTGTTGGTC 2280
CAAAATGCGA ACCCAGATTG TAAGACTATT TTAAAAGCAT TGGGACCAGG AGCGACACTA 2340
GAAGAAATGA TGACAGCATG TCAGGGAGTG GGGGGACCCG GCCATAAAGC AAGAGTTTTG 2400
GCTGAAGCAA TGAGCCAAGT AACAAATCCA GCTACCATAA TGATACAGAA AGGCAATTTT 2460

AGGAACCAAA GAAAGACTGT TAAGTGTTTC AATTGTGGCA AAGAAGGGCA CATAGCCAAA 2520

AATTGCAGGG CCCCTAGGAA AAAGGGCTGT TGGAAATGTG GAAAGGAAGG ACACCAAATG 2580

AAAGATTGTA CTGAGAGACA GGCTAATTTT TTAGGGAAGA TCTGGCCTTC CCACAAGGGA 2640

AGGCCAGGGA ATTTTCTTCA GAGCAGACCA GAGCCAACAG CCCCACCAGA AGAGAGCTTC 2700

AGGTTTGGGG AAGAGACAAC AACTCCCTCT CAGAAGCAGG AGCCGATAGA CAAGGAACTG 2760

TATCCTTTAG CTTCCCTCAG ATCACTCTTT GGCAGCGACC CCTCGTCACA ATGA 2814

(2) INFORMATION FOR SEQ ID NO:58:

(i) SEQUENCE CHARACTERISTICS:
(A) LENGTH: 938 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:58:

```
Met Lys Lys Thr Ala Ile Ala Ile Ala Val Ala Leu Ala Gly Phe Ala
1               5                   10                  15

Thr Val Ala Gln Ala Ala Asn Leu Ala Glu Glu Ala Phe Asp Leu Trp
            20                  25                  30

Asn Glu Cys Ala Lys Ala Cys Val Leu Asp Leu Lys Asp Gly Val Arg
        35                  40                  45

Ser Ser Arg Met Ser Val Asp Pro Ala Ile Ala Asp Thr Asn Gly Gln
    50                  55                  60

Gly Val Leu His Tyr Ser Met Val Leu Glu Gly Gly Asn Asp Ala Leu
65                  70                  75                  80

Lys Leu Ala Ile Asp Asn Ala Leu Ser Ile Thr Ser Asp Gly Leu Thr
                85                  90                  95

Ile Arg Leu Glu Gly Gly Val Glu Pro Asn Lys Pro Val Arg Tyr Ser
            100                 105                 110

Tyr Thr Arg Gln Ala Arg Gly Ser Trp Ser Leu Asn Trp Leu Val Pro
        115                 120                 125
```

```
Ile Gly His Glu Lys Pro Ser Asn Ile Lys Val Phe Ile His Glu Leu
    130                 135                 140

Asn Ala Gly Asn Gln Leu Ser His Met Ser Pro Ile Tyr Thr Ile Glu
145                 150                 155                 160

Met Gly Asp Glu Leu Leu Ala Lys Leu Ala Arg Asp Ala Thr Phe Phe
                165                 170                 175

Val Arg Ala His Glu Ser Asn Glu Met Gln Pro Thr Leu Ala Ile Ser
            180                 185                 190

His Ala Gly Val Ser Val Val Met Ala Gln Thr Gln Pro Arg Arg Glu
        195                 200                 205

Lys Arg Trp Ser Glu Trp Ala Ser Gly Lys Val Leu Cys Leu Leu Asp
    210                 215                 220

Pro Leu Asp Gly Val Tyr Asn Tyr Leu Ala Gln Gln Arg Cys Asn Leu
225                 230                 235                 240

Asp Asp Thr Trp Glu Gly Lys Ile Tyr Arg Val Leu Ala Gly Asn Pro
                245                 250                 255

Ala Lys His Asp Leu Asp Ile Lys Pro Thr Val Ile Ser His Arg Leu
            260                 265                 270

His Phe Pro Glu Gly Gly Ser Leu Ala Ala Leu Thr Ala His Gln Ala
        275                 280                 285

Cys His Leu Pro Leu Glu Thr Phe Thr Arg His Arg Gln Pro Arg Gly
    290                 295                 300

Trp Glu Gln Leu Glu Gln Cys Gly Tyr Pro Val Gln Arg Leu Val Ala
305                 310                 315                 320

Leu Tyr Leu Ala Ala Arg Leu Ser Trp Asn Gln Val Asp Gln Val Ile
                325                 330                 335

Arg Asn Ala Leu Ala Ser Pro Gly Ser Gly Gly Asp Leu Gly Glu Ala
            340                 345                 350

Ile Arg Glu Gln Pro Glu Gln Ala Arg Leu Ala Leu Thr Leu Ala Ala
        355                 360                 365

Ala Glu Ser Glu Arg Phe Val Arg Gln Gly Thr Gly Asn Asp Glu Ala
    370                 375                 380
```

Gly Ala Ala Asn Ala Asp Val Val Ser Leu Thr Cys Pro Val Ala Ala
385 390 395 400

Gly Glu Cys Ala Gly Pro Ala Asp Ser Gly Asp Ala Leu Leu Glu Arg
405 410 415

Asn Tyr Pro Thr Gly Ala Glu Phe Leu Gly Asp Gly Gly Asp Val Ser
420 425 430

Phe Ser Thr Arg Gly Met Gly Ala Arg Ala Ser Val Leu Ser Gly Gly
435 440 445

Glu Leu Asp Lys Trp Glu Lys Ile Arg Leu Arg Pro Gly Gly Lys Lys
450 455 460

Gln Tyr Lys Leu Lys His Ile Val Trp Ala Ser Arg Glu Leu Glu Arg
465 470 475 480

Phe Ala Val Asn Pro Gly Leu Leu Glu Thr Ser Glu Gly Cys Arg Gln
485 490 495

Ile Leu Gly Gln Leu Gln Pro Ser Leu Gln Thr Gly Ser Glu Glu Leu
500 505 510

Arg Ser Leu Tyr Asn Thr Ile Ala Val Leu Tyr Cys Val His Gln Arg
515 520 525

Ile Asp Val Lys Asp Thr Lys Glu Ala Leu Asp Lys Ile Glu Glu Glu
530 535 540

Gln Asn Lys Ser Lys Lys Lys Ala Gln Gln Ala Ala Ala Asp Thr Gly
545 550 555 560

Asn Asn Ser Gln Val Ser Gln Asn Tyr Pro Ile Val Gln Asn Leu Gln
565 570 575

Gly Gln Met Val His Gln Ala Ile Ser Pro Arg Thr Leu Asn Ala Trp
580 585 590

Val Lys Val Val Glu Glu Lys Ala Phe Ser Pro Glu Val Ile Pro Met
595 600 605

Phe Ser Ala Leu Ser Glu Gly Ala Thr Pro Gln Asp Leu Asn Thr Met
610 615 620

Leu Asn Thr Val Gly Gly His Gln Ala Ala Met Gln Met Leu Lys Glu
625 630 635 640

Thr Ile Asn Glu Glu Ala Ala Glu Trp Asp Arg Leu His Pro Val His
645 650 655

Ala Gly Pro Ile Ala Pro Gly Gln Met Arg Glu Pro Arg Gly Ser Asp
660 665 670

Ile Ala Gly Thr Thr Ser Thr Leu Gln Glu Gln Ile Gly Trp Met Thr
675 680 685

His Asn Pro Pro Ile Pro Val Gly Glu Ile Tyr Lys Arg Trp Ile Ile
690 695 700

Leu Gly Leu Asn Lys Ile Val Arg Met Tyr Ser Pro Thr Ser Ile Leu
705 710 715 720

Asp Ile Arg Gln Gly Pro Lys Glu Pro Phe Arg Asp Tyr Val Asp Arg
725 730 735

Phe Tyr Lys Thr Leu Arg Ala Glu Gln Ala Ser Gln Glu Val Lys Asn
740 745 750

Trp Met Thr Glu Thr Leu Leu Val Gln Asn Ala Asn Pro Asp Cys Lys
755 760 765

Thr Ile Leu Lys Ala Leu Gly Pro Gly Ala Thr Leu Glu Glu Met Met
770 775 780

Thr Ala Cys Gln Gly Val Gly Gly Pro Gly His Lys Ala Arg Val Leu
785 790 795 800

Ala Glu Ala Met Ser Gln Val Thr Asn Pro Ala Thr Ile Met Ile Gln
805 810 815

Lys Gly Asn Phe Arg Asn Gln Arg Lys Thr Val Lys Cys Phe Asn Cys
820 825 830

Gly Lys Glu Gly His Ile Ala Lys Asn Cys Arg Ala Pro Arg Lys Lys
835 840 845

Gly Cys Trp Lys Cys Gly Lys Glu Gly His Gln Met Lys Asp Cys Thr
850 855 860

Glu Arg Gln Ala Asn Phe Leu Gly Lys Ile Trp Pro Ser His Lys Gly
865 870 875 880

Arg Pro Gly Asn Phe Leu Gln Ser Arg Pro Glu Pro Thr Ala Pro Pro
885 890 895

Glu Glu Ser Phe Arg Phe Gly Glu Glu Thr Thr Thr Pro Ser Gln Lys
900 905 910

Gln Glu Pro Ile Asp Lys Glu Leu Tyr Pro Leu Ala Ser Leu Arg Ser
915 920 925

Leu Phe Gly Ser Asp Pro Ser Ser Gln Xaa
930 935

## Claims

1. A recombinant DNA segment comprising a nucleotide sequence coding for a hybrid protein comprising a modified Pseudomonas exotoxin and a polypeptide that is exogenous to an antigen-presenting cell, said hybrid capable of being at least partially presented on an antigen-presenting cell surface.

2. A recombinant DNA segment comprising a nucleotide sequence coding for a hybrid protein comprising a modified Pseudomonas exotoxin and a polypeptide of viral origin, said hybrid capable of being at least partially presented on an antigen-presenting cell surface.

3. A recombinant DNA segment comprising a nucleotide sequence coding for a hybrid protein comprising a modified Pseudomonas exotoxin and a polypeptide of viral origin, said hybrid being capable of being internalized by an antigen-presenting cell and further capable of being at least partially presented on the surface of Said antigen-presenting cell.

4. A recombinant DNA segment comprising a nucleotide sequence coding for a hybrid protein comprising a modified Pseudomonas exotoxin and a polypeptide of viral origin, said hybrid capable of being internalized by an antigen-presenting cell and further capable of being processed for at least partial presentation on the surface of said antigen-presenting cell, sufficiently to elicit an immune response by cytotoxic T lymphocytes.

5. A transformant harboring a recombinant DNA segment comprising a nucleotide sequence coding for a hybrid protein comprising a modified Pseudomonas exotoxin and a polypeptide that is exogenous to an antigen-presenting cell, said hybrid capable of eliciting an immune response by cytotoxic T lymphocytes.

6. A transformant harboring a recombinant DNA segment comprising a nucleotide sequence coding for a hybrid protein comprising a modified Pseudomonas exotoxin and a polypeptide that is exogenous to an antigen-presenting cell said hybrid capable of being at least partially presented on an antigen-presenting cell surface.

7. A transformant harboring a recombinant DNA segment comprising a nucleotide sequence coding for a hybrid protein comprising a modified Pseudomonas exotoxin and a polypeptide of viral origin, said hybrid capable of being at least partially presented on an antigen-presenting cell surface.

8. A transformant harboring a recombinant DNA segment comprising a nucleotide sequence coding for a hybrid protein comprising a modified Pseudomonas exotoxin and a polypeptide of viral origin, said hybrid capable of being internalized by an antigen-presenting cell, and further capable of being at least partially presented on the surface of said antigen-presenting cell.

9. The recombinant DNA segment as claimed in any one of claims 1 to 4, wherein said modified Pseudomonas exotoxin lacks a functioning ADP ribosylating domain.

10. The recombinant DNA segment as claimed in claim 2, wherein said polypeptide of viral origin is a viral protein fragment comprising the matrix protein of influenza A virus.

11. The recombinant DNA segment as claimed in claim 10, wherein said viral protein fragment comprises residues 57 to 68 of the matrix protein of influenza A virus.

12. The recombinant DNA segment as claimed in claim 2, wherein said polypeptide of viral origin is a viral protein fragment comprising the gag protein of human immunodeficiency virus-1.

13. The recombinant DNA segment as claimed in claim 2, wherein said polypeptide of viral origin is a viral protein fragment comprising the nucleoprotein of influenza A virus.

14. The transformant as claimed in claim 5 wherein said modified Pseudomonas exotoxin lacks a functioning ADP ribosylating domain.

15. The transformant as claimed in claim 7, wherein said polypeptide of viral origin is a viral protein fragment comprising the viral matrix protein of influenza A virus.

16. The transformant as claimed in claim 15, wherein said viral protein fragment comprises residues 57 to 68 of the matrix protein of influenza A virus.

17. The transformant as claimed in claim 7, wherein said polypeptide of viral origin is a viral protein fragment which is sufficiently specific to bind to HLA-2.

18. The transformant as claimed in claim 7, wherein said polypeptide of viral origin is a viral protein fragment comprising the nucleoprotein of influenza A virus.

19. The transformant as claimed in claim 7, wherein said polypeptide of viral origin is a viral protein fragment comprising the gag protein of human immunodeficiency virus-1.

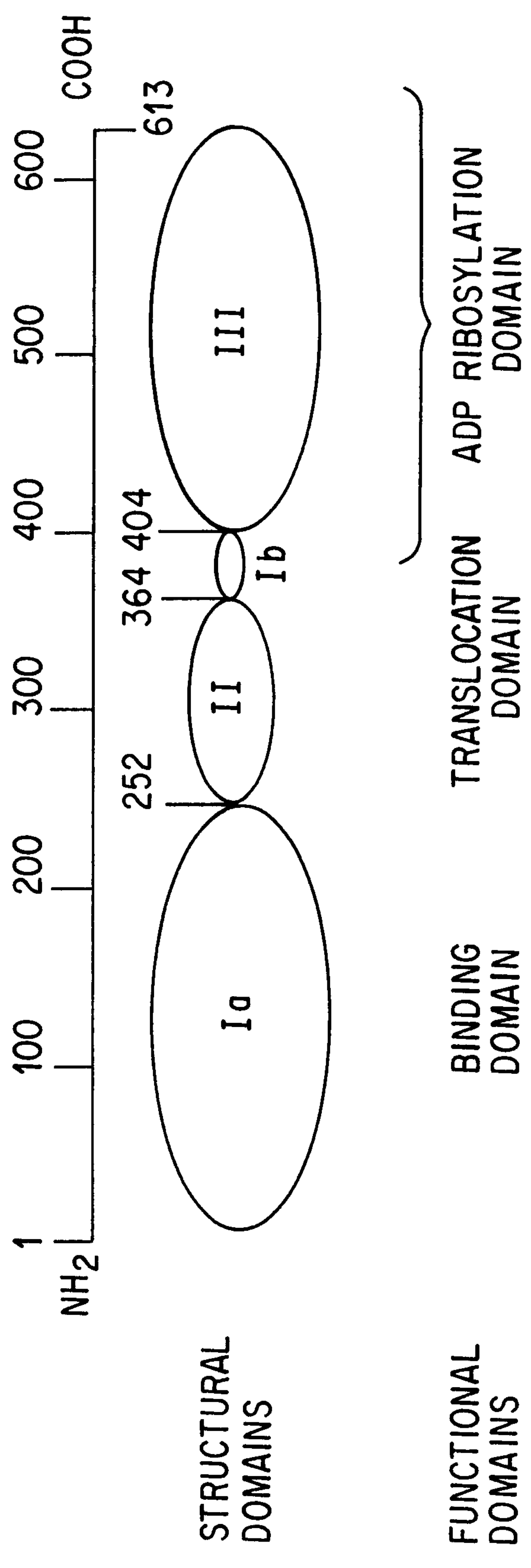
STRUCTURAL DOMAINS
FUNCTIONAL DOMAINS
$NH_2$
COOH
1
100
200
300
400
500
600
252
364
404
613
Ia
II
Ib
III
BINDING DOMAIN
TRANSLOCATION DOMAIN
ADP RIBOSYLATION DOMAIN

FIG. 1

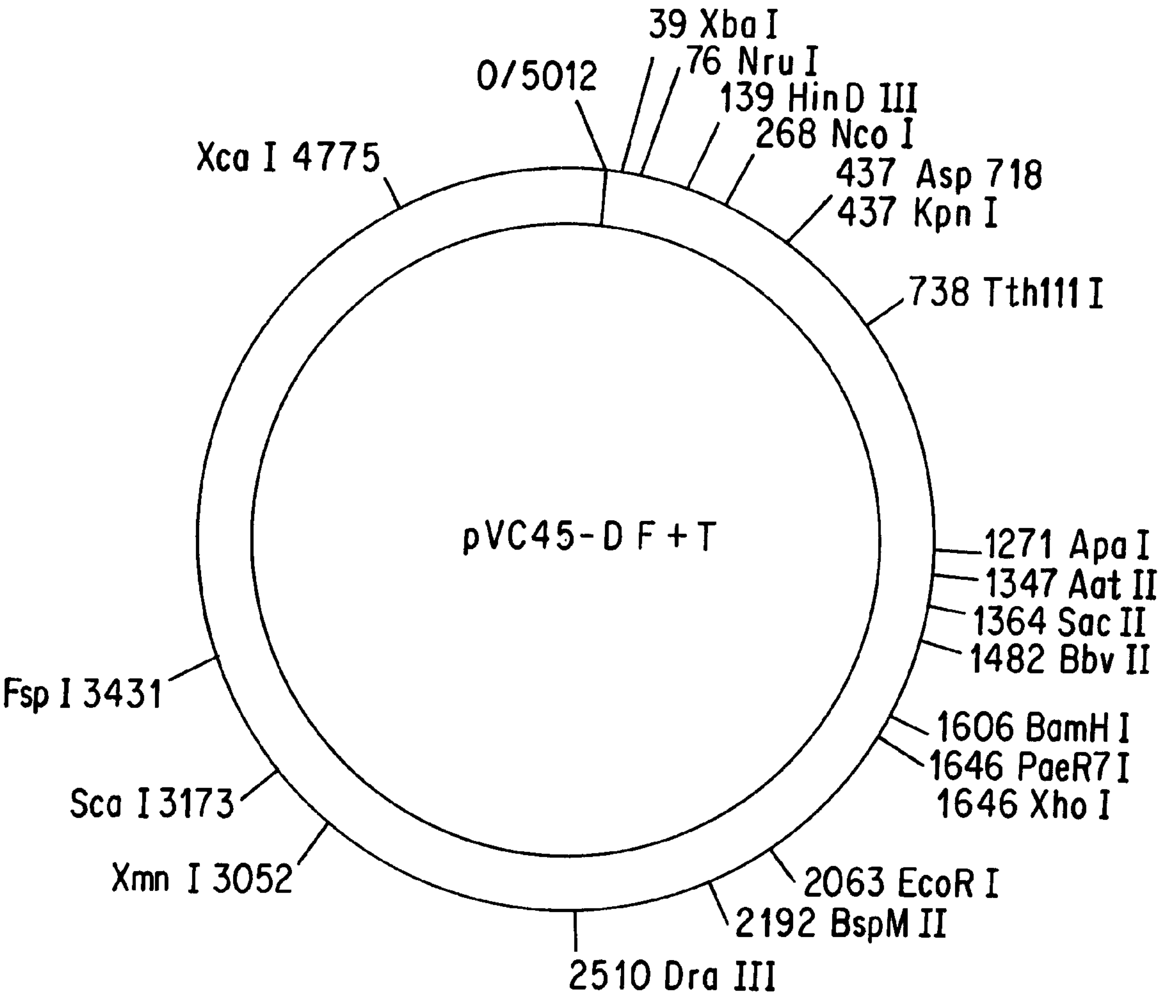
39 Xba I
76 Nru I
139 HinD III
268 Nco I
437 Asp 718
437 Kpn I
738 Tth111 I
1271 Apa I
1347 Aat II
1364 Sac II
1482 Bbv II
1606 BamH I
1646 PaeR7 I
1646 Xho I
2063 EcoR I
2192 BspM II
2510 Dra III
Xmn I 3052
Sca I 3173
Fsp I 3431
Xca I 4775
0/5012
pVC45-D F + T

F I G. 2

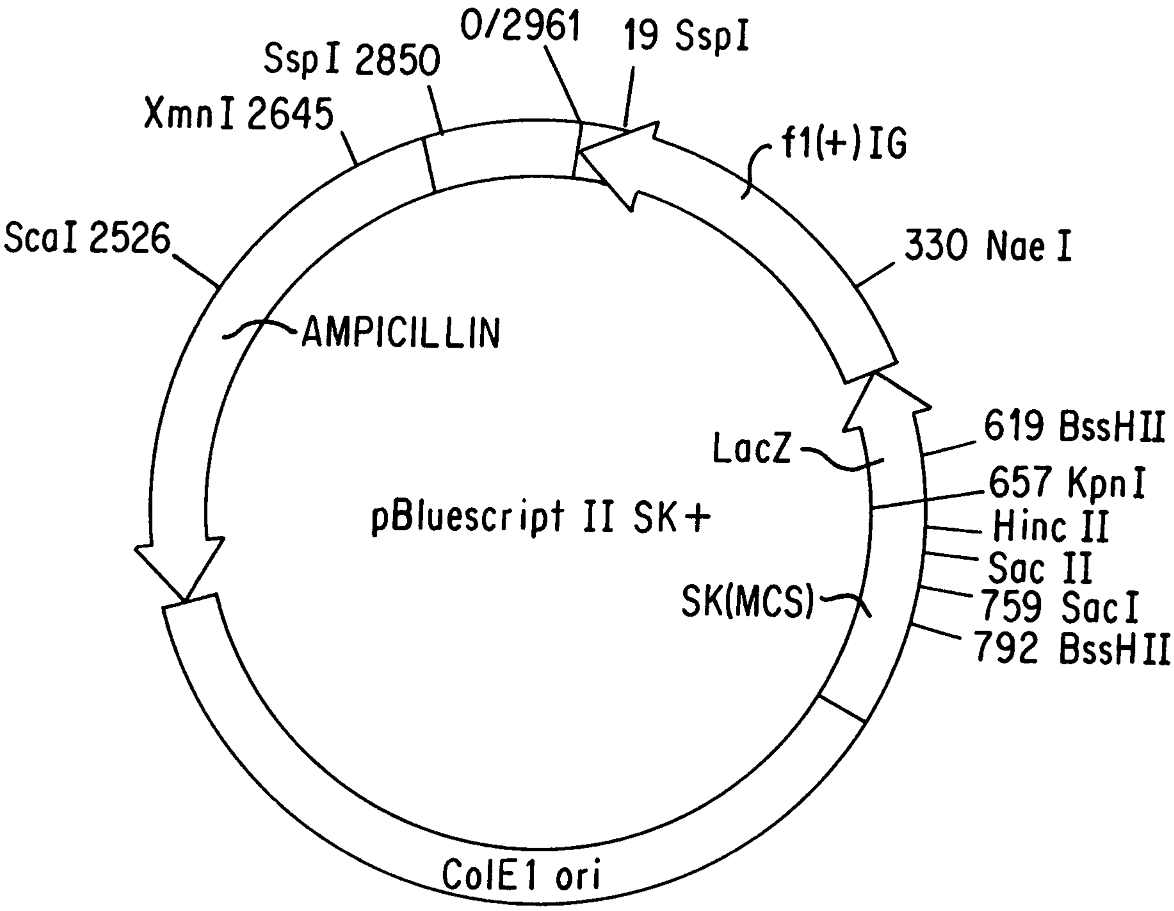
0/2961
19 SspI
SspI 2850
XmnI 2645
f1(+)IG
ScaI 2526
330 Nae I
AMPICILLIN
619 BssHII
LacZ
657 KpnI
pBluescript II SK+
Hinc II
Sac II
SK(MCS)
759 SacI
792 BssHII
ColE1 ori

FIG. 3

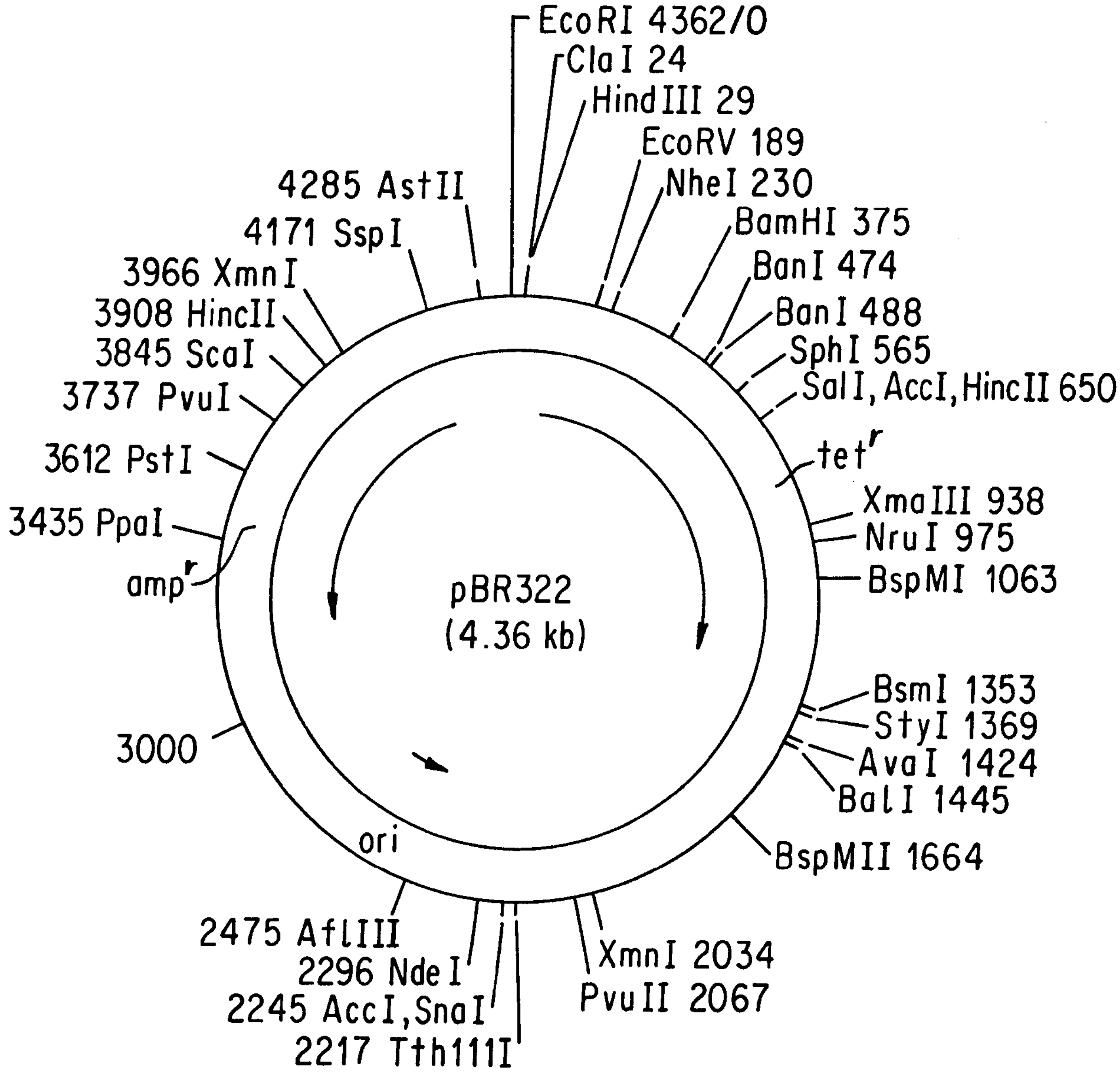
EcoRI 4362/0
ClaI 24
HindIII 29
EcoRV 189
NheI 230
BamHI 375
BanI 474
BanI 488
SphI 565
SalI, AccI, HincII 650
tet^r
XmaIII 938
NruI 975
BspMI 1063
BsmI 1353
StyI 1369
AvaI 1424
BalI 1445
BspMII 1664
XmnI 2034
PvuII 2067
2217 Tth111I
2245 AccI, SnaI
2296 NdeI
2475 AflIII
ori
3000
amp^r
3435 PpaI
3612 PstI
3737 PvuI
3845 ScaI
3908 HincII
3966 XmnI
4171 SspI
4285 AstII
pBR322
(4.36 kb)

FIG. 4

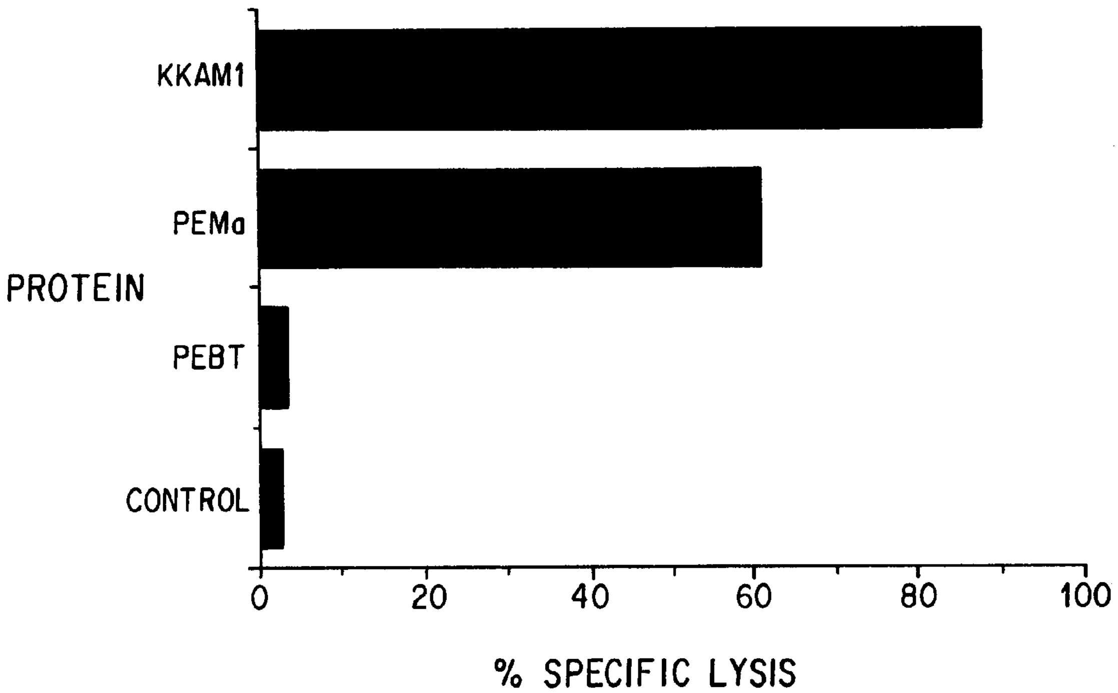
KKAM1
PEMa
PROTEIN
PEBT
CONTROL
0
20
40
60
80
100
% SPECIFIC LYSIS

FIG. 5

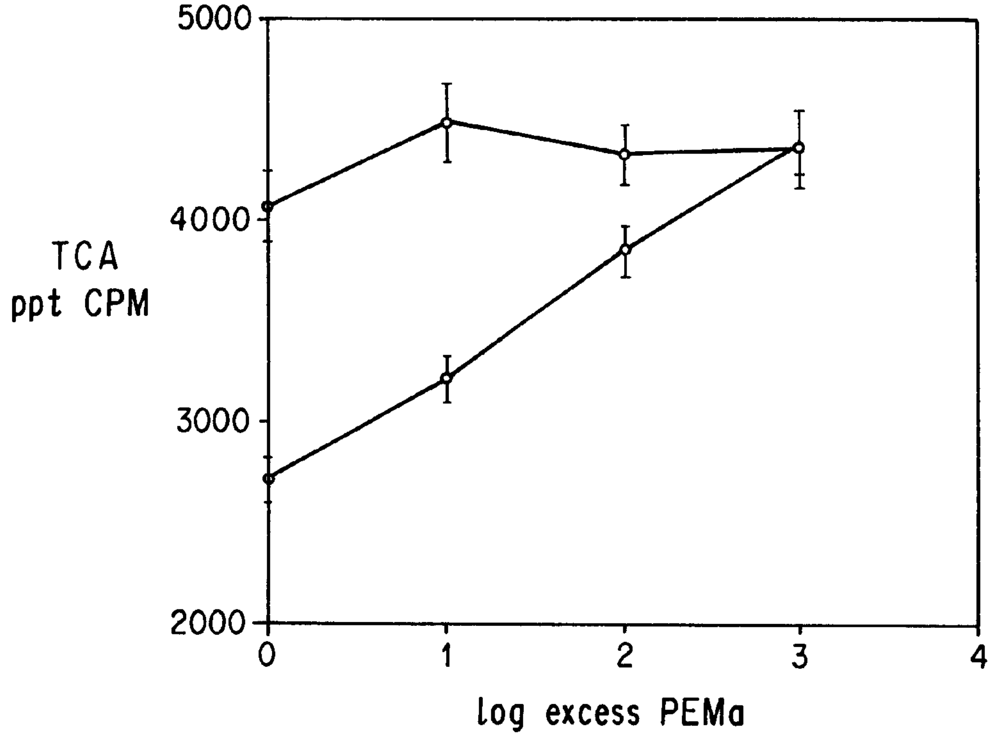
5000
4000
3000
2000
TCA
ppt CPM
0
1
2
3
4
log excess PEMa

FIG. 6

European Patent Office

# EUROPEAN SEARCH REPORT

Application Number

EP 92310067.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A - 0 261 671 (I. PASTAN et al.) * Claims 1,6,7 * -- | 1-4,9-13 | C 12 N 15/62<br>C 12 N 5/10 |
| A | EP - A - 0 431 327 (HOECHST AG) * Claims 1,5 * -- | 1-19 | |
| A | CHEMICAL ABSTRACTS, vol. 108, no. 7, February 15, 1988 Columbus, Ohio, USA T. ZEHAVI-WILLNER "Induction of murine cytolytic T lymphocytes by Pseudomonas aeruginosa exotoxin A", page 564, column 2, abstract-no. 54 194h & Infect. Immun. 1988, 56(1), 213-18 ---- | 1-19 | |

TECHNICAL FIELDS SEARCHED (Int. Cl.5)

C 12 N 5/00
C 12 N 15/00
A 61 K 37/00
A 61 K 39/00
C 07 K 15/00
C 12 P 21/00

The present search report has been drawn up for all claims

| Place of search | Date of completion of the search | Examiner |
|---|---|---|
| VIENNA | 19-02-1993 | SCHARF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)